# EUROPEAN PATENT SPECIFICATION

(11) **EP 3 632 326 B1**
(45) Date of publication and mention of the grant of the patent: **05.07.2023**
(21) Application number: 16925744.1
(22) Date of filing: 30.12.2016
(51) Int. Cl.: A61B 6/03, A61B 6/00, A61B 6/04

(54) **MEDICAL IMAGING SYSTEM AND METHOD**
SYSTEM UND VERFAHREN FÜR MEDIZINISCHE BILDGEBUNG
SYSTÈME ET PROCÉDÉ D'IMAGERIE MÉDICALE

(43) Date of publication of application: 08.04.2020
(73) Proprietor: Shanghai United Imaging Healthcare Co., Ltd., Shanghai 201807 (CN)
(72) Inventor: LI, Hongdi, Houston Texas 77054 (US); LIU, Weiping, Shanghai 201807 (CN); GU, Xiaoyue, Shanghai 201807 (CN)
(74) Representative: Wang, Bo
(86) International application number: PCT/CN2016/113598
(87) International publication number: WO 2018/120061

(56) References cited:
- CN-A- 1 919 147
- CN-A- 103 815 924
- CN-A- 104 146 725
- CN-A- 105 814 454
- US-A1- 2005 267 350
- US-A1- 2010 128 956
- US-A1- 2010 128 956
- US-A1- 2012 161 014
- US-A1- 2014 316 258

## Description

### TECHNICAL FIELD

The present disclosure generally relates to a system and method for medical imaging, and more particularly, a positron emission tomography (PET) system and method for multi-organ simultaneous dynamic acquisition.

### BACKGROUND

Among medical scanning imaging, PET scanning can be used to image the metabolism of tissues of human body, especially for early diagnosis and therapeutic efficacy assessment of cancer, coronary heart disease, brain disease, and many other diseases. Dynamic acquisition imaging can provide information on the metabolism of human organs over time. However, the axial field of view of scanning of a commercial PET scanner is relatively small (e.g., usually 16-22cm). It is difficult to compare the metabolism of multiple organs since the commercial PET scanner cannot perform dynamic imaging of multiple organs simultaneously.

If the PET scanner images multiple organs respectively, the decay of an agent will make the imaging results of different organs not be comparable. For an agent with a shorter half-life, this effect will be greater. The manufacturing cost of a PET scanner with a wide axial field of view of scanning is very high. Therefore, it is needed to design a high cost-effective PET system which can proceed multi-organ simultaneous dynamic acquisition.

Systems for medical imaging comprising multiple PET scanners are known from documents US 2014/316258 A1, US 2010/128956 A1, and US 2012/161014 A1.

### SUMMARY

A system for medical imaging as defined in claim 1 is provided.

In some embodiments, the first PET scanner may include a first PET detector ring and the second PET scanner may include a second PET detector ring.

In some embodiments, a radius of the first PET detector ring may be same as a radius of the second PET detector ring.

In some embodiments, a radius of the first PET detector ring may be different from a radius of the second PET detector ring.

In some embodiments, the first PET scanner and the second PET scanner may be mounted on the driving device, and the driving device may drive the first PET scanner and the second PET scanner to move along an axial direction.

In some embodiments, the system may further include a CT scanner configured to obtain CT scanning data. The processor may further generate a scout image based on the CT scanning data.

In some embodiments, the scout image may include a first identifier and a second identifier. The controller may determine the first scanning location and the second scanning location by obtaining a location of the first identifier and a location of the second identifier.

In some embodiments, the controller may determine a distance for moving the first PET scanner and a distance for moving the second PET scanner by obtaining the first scanning location and the second scanning location.

In some embodiments, the controller may determine the first scanning location and the second scanning location by obtaining a height of a patient.

In some embodiments, the first PET scanner may obtain the first scanning data at one or more first time points. The second PET scanner may obtain the second scanning data at one or more second time points. The processor may perform dynamic imaging on the first scanning area based on the first scanning data at the one or more first time points. The processor may perform dynamic imaging on the second scanning area based on the second scanning data at the one or more second time points.

In some embodiments, the processor may determine metabolism of an agent in the first scanning area and the second scanning area based on the dynamic imaging. The metabolism may changes over time.

In some embodiments, the processor may generate the first image of the first scanning area based on the first scanning data and generate the second image of the second scanning area based on the second scanning data simultaneously.

In some embodiments, the processor may generate a third image of a third scanning area based on scanning data obtained by the first PET scanner and the second PET scanner. The third scanning area may be between the first scanning area and the second scanning area.

In some embodiments, the processor may generate the third image of the third scanning area based on the scanning data obtained by the first PET scanner and the second PET scanner. The processor may further obtain third scanning data through both the first PET scanner and the second PET scanner, and generate the third image of the third scanning area based on the third scanning data.

In some embodiments, the processor may generate a full image by stitching images of the first scanning area, the second scanning area, and the third scanning area that are generated based on the scanning data obtained by the first PET scanner and the second PET scanner.

In some embodiments, the first PET scanner may obtain a first scanning parameter and scan the first scanning area according to the first scanning parameter. The second PET scanner may obtain a second scanning parameter and scan the second scanning area according to the second scanning parameter. The first scanning parameter may be same as the second scanning parameter.

In some embodiments, the first PET scanner may obtain a first scanning parameter and scan the first scanning area according to the first scanning parameter. The second PET scanner obtains a second scanning parameter and scan the second scanning area according to the second scanning parameter. The first scanning parameter may be different from the second scanning parameter.

In some embodiments, the processor may obtain a first reconstruction parameter and generate the first image of the first scanning area according to the first reconstruction parameter. The processor may obtain a second reconstruction parameter and generate the second image of the second scanning area according to the second reconstruction parameter. The first reconstruction parameter may be same as the second reconstruction parameter.

In some embodiments, the processor may obtain a first reconstruction parameter and generate the first image of the first scanning area according to the first reconstruction parameter. The processor may obtain a second reconstruction parameter and generate the second image of the second scanning area according to the second reconstruction parameter. The first reconstruction parameter may be different from the second reconstruction parameter.

In some embodiments, a field of view (FOV) of scanning along an axial direction or a radial direction of the second PET scanner may be different from an FOV of scanning along an axial direction or a radial direction of the first PET scanner.

In some embodiments, an FOV of scanning along an axial direction or a radial direction of the second PET scanner may be same as an FOV of scanning along an axial direction or a radial direction of the first PET scanner.

A system for medical imaging as defined in claim 12 is provided.

A method for medical imaging as defined in claim 13 is provided.

In some embodiments, the method may further include scanning the first scanning area corresponding to the first scanning location according to the first PET scanning parameter by the first PET scanner, and the scanning the second scanning area corresponding to the second scanning location according to the second PET scanning parameter is performed by the second PET scanner.

In some embodiments, the method may further include moving the first PET scanner and the second PET scanner to the first scanning location and the second scanning location based on the first scanning location and the second scanning location, respectively.

In some embodiments, the method may further include obtaining the first scanning data at one or more first time points; obtaining the second scanning data at one or more second time points; and performing dynamic imaging on the first scanning area based on the first scanning data at the one or more first time points; and performing dynamic imaging on the second scanning area based on the second scanning data at one or more second time points.

In some embodiments, the method may further include determining metabolism of an agent in the first scanning area and the second scanning area based on the dynamic imaging, wherein the metabolism may change over time.

Additional features will be set forth in part in the description which follows, and in part will become apparent to those skilled in the art upon examination of the following and the accompanying drawings or may be learned by production or operation of the examples. The features of the present disclosure may be realized and attained by practice or use of various aspects of the methodologies, instrumentalities, and combinations set forth in the detailed examples discussed below.

### BRIEF DESCRIPTION OF THE DRAWINGS

In order to illustrate the technical solutions related to the embodiments of the present disclosure, brief introduction of the drawings referred to the description of the embodiments is provided below. Obviously, drawings described below are only some examples or embodiments of the present disclosure. Those having ordinary skills in the art, without further creative efforts, may apply the present disclosure to other similar scenarios according to these drawings. Unless stated otherwise or obvious from the context, the same reference numeral in the drawings refers to the same structure and operation.
FIG. 1 illustrates an exemplary imaging system according to some embodiments of the present disclosure;
FIG. 2A is a block diagram of an exemplary imaging apparatus according to some embodiments of the present disclosure;
FIG. 2B is a block diagram of an exemplary computer equipment according to some embodiments of the present disclosure;
FIG. 3 is a block diagram of an exemplary imaging system according to some embodiments of the present disclosure;
FIG. 4 is a flowchart illustrating an exemplary process for generating a scanning image according to some embodiments of the present disclosure;
FIG. 5 is a block diagram of an exemplary scanning module according to some embodiments of the present disclosure;
FIG. 6 is a flowchart illustrating an exemplary process for setting a PET scanning parameter according to some embodiments of the present disclosure;
FIG. 7 is a block diagram of an exemplary scanning module according to some embodiments of the present disclosure;
FIG. 8 is a flowchart illustrating an exemplary process for obtaining PET scanning data according to some embodiments of the present disclosure;
FIG. 9 is a flowchart illustrating an exemplary process for processing the obtained PET scanning data according to some embodiments of the present disclosure;
FIG. 10 is a block diagram of an exemplary scanning module according to some embodiments of the present disclosure;
FIG. 11 is a block diagram of an exemplary scanning module according to some embodiments of the present disclosure;
FIG. 12 is a block diagram of an exemplary scanning module according to some embodiments of the present disclosure; and
FIG. 13 is a block diagram of an exemplary scanning module according to some embodiments of the present disclosure.

### DETAILED DESCRIPTION

In order to illustrate the technical solutions related to the embodiments of the present disclosure, brief introduction of the drawings referred to the description of the embodiments is provided below. Obviously, drawings described below are only some examples or embodiments of the present disclosure. Those having ordinary skills in the art, without further creative efforts, may apply the present disclosure to other similar scenarios according to these drawings. Unless stated otherwise or obvious from the context, the same reference numeral in the drawings refers to the same structure and operation.

**As** described in the specification and claims, the singular forms "a," "an," and "the" may be intended to include the plural forms as well, unless the context clearly indicates otherwise. It will be further understood that the terms "comprises," "comprising," "includes," and/or "including" when used in this disclosure, specify the presence of stated steps and elements, but do not preclude the presence or addition of one or more other steps and elements. In addition, the term "based on" may include "at least in part based on." The term "one embodiment" may include "at least one embodiment." The term "another embodiment" may include "at least one other embodiment." The definitions of other terms may be provide in the following description of the present disclosure.

**Some** modules of the system may be referred to in various ways according to some embodiments of the present disclosure, however, any number of different modules may be used and operated in an electric control equipment. These modules are intended to be illustrative, not intended to limit the scope of the present disclosure. Different modules may be used in different aspects of the system and method.

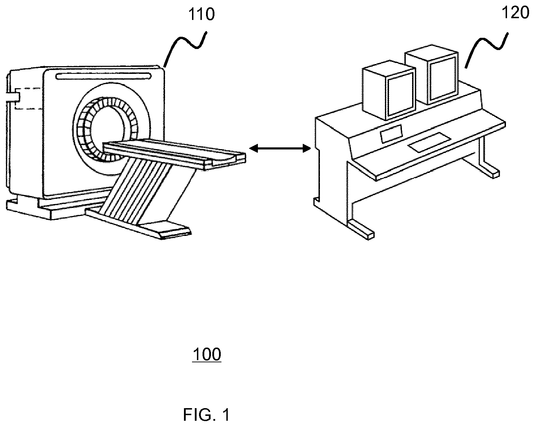

FIG. 1 is a schematic diagram of an imaging system 100 according to some embodiments of the present disclosure. The imaging system may scan one or more target objects and generate one or more corresponding images based on the scanning data. In some embodiments, the imaging system 100 may be a system for medical imaging. The imaging system may include a imaging apparatus 110 and a computing device 120. The imaging apparatus 110 may scan the target object and obtain corresponding scanning data. The target object may be a human body, an animal, an abiotic object, or the like. For example, the target object may include an organ, a vertebrae, a bone, tissue, a blood vessel of the human or animal, or an abiotic sample for calibrating system parameters. The imaging apparatus 110 may be or include a scanner. The scanner may include a positron emission tomography (PET) scanner, a computed tomography (CT) scanner, a magnetic resonance imaging (MRI) scanner, a b-scan ultrasonography scanner, a thermal texture map (TTM) scanner, or the like, or any combination thereof. In some embodiments, imaging apparatus 110 may include a plurality of PET scanners and a CT scanner.

In some embodiments, the imaging apparatus 110 may further include a scanning bed and a driving device (not shown in FIG. 1). The scanning bed may be configured to support the target object. The driving device may be configured to drive one or more scanners to move. A detailed embodiment of the imaging apparatus 110 may be found in other parts of the present disclosure.

The computing device 120 may be associated with the imaging apparatus 110. In some embodiments, the computing device 120 may receive scanning data obtained by the imaging apparatus 110 and generate a corresponding image based on the scanning data. In some embodiments, the computing device 120 may include a console. A user (e.g., a doctor, an imaging technician) may control the imaging apparatus 110 to scan the object (e.g., a patient) through the console. A detailed embodiment of the computing device 120 may be found in other parts of the present disclosure.

In some embodiments, the imaging system 100 may connect to a network through the computing device 120. The network may include a wired connection or wireless connection. In some embodiments, the network may be a single network or a combination of multiple networks. For example, the network may include a local area network, a public network, a private network, a wireless local area network, a virtual network, a metropolitan area network, a public switched telephone network, or the like, or any combination thereof. The network may include multiple network access points, for example, a wired or wireless access point, a base station or network switching points. Each component of the imaging system 100 may connect to the network through the access points to achieve information interaction. For example, the imaging system 100 may connect to a public medical system through the network and obtain historical medical information of a patient or synchronize medical data of the patient.

In some embodiments, the imaging system 100 may connect to and communicate with an external server, a database, or a terminal device through the network. The server or database may store information and retrieve information. The server may include a file server, a database server, a file transfer protocol (FTP) server, an application server, a proxy server, a mail server, or the like, or any combination thereof. In some embodiments, the server may be a cloud server. The database may include a hierarchical database, a network database, a relational database, or the like, or any combination thereof. The terminal device may be configured to receive information outputted by the imaging system 100. The information may include a disease diagnosis report, a prescription issued by a physician, or other information that may be requested by a user. In some embodiments, the terminal device may include a laptop, a mobile phone, a tablet, a console, an intelligent wear device (e.g., a smart watch), or the like, or any combination thereof.

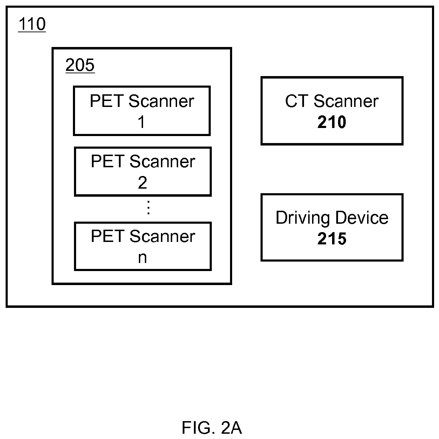

FIG. 2A is a schematic diagram of an exemplary imaging apparatus according to some embodiments of the present disclosure. The imaging apparatus 110 may include a PET scanning component 205, a CT scanner 210, and a driving device 215. The PET scanning component 205 may include one or more PET scanners, for example, PET scanner 1, PET scanner 2, ... , PET scanner n. In some embodiments, the one or more PET scanners may be mutually independent. In some embodiments, at least part of the one or more PET scanners may be associated with each other.

The PET scanner may perform PET scanning on the target object and obtain corresponding PET scanning data. The PET scanner may include one or more detector rings (e.g., an annular detector). The detector ring may include a plurality of detector elements arranged along a circumferential direction. In some embodiments, the number of the detector elements may relate to the accuracy and spatial resolution of the detector. The shape of the detector element may be wedge, square or other shapes. In some embodiments, the detector element may be wedge-shaped. The wedge-shaped detector element may be tightly fitted to form a complete detector ring. In some embodiments, the detector element may include a crystal and a photomultiplier. In some embodiments, the detector element may include a crystal and a silicon photomultiplier (SiPM). A signal within a detection range of the detector ring may be received by the crystal and amplified by the photomultiplier for further processing. In some embodiments, a PET scanner may include a plurality of detector rings. The plurality of detector rings may be arranged along the axial direction, so that the PET scanner may have a wider axial scanning range (an axial field of view (axial FOV) of scanning). In some embodiments, a plurality of PET scanners of the PET scanning component 205 (e.g., the PET scanner 1, the PET scanner 2, ... , the PET scanner n) may have same or different FOV(s) of scanning along the axial direction or the radial direction. For example, the PET scanner 1 may include two detector rings. The PET scanner 2 may include three or more detector rings. Compared with the PET scanner 1, the PET scanner 2 may have a wider FOV of scanning along the axial direction. In some embodiments, the FOV of scanning in the axial direction or the radial direction of the PET scanner may depend on a diameter of the detector ring, the number of the detector ring, a shape of the internal detector elements, the number of the detector elements, or a type of the detector elements.

Before PET scanning, one or more agents may be introduced into the target object. In some embodiments, the agent may include a substance required for the metabolism of an organism. For example, the agent may include glucose, protein, nucleic acid, fatty acid, or the like. In some embodiments, the agent may be labeled with one or more radionuclides. The radionuclide may include F18, C11, etc. In some embodiments, the agent may be fluorodeoxyglucose (FDG) labeled with F18. In some embodiments, a positron of the radionuclide may be combined with an electron at a corresponding position, producing a pair of photons emitted in the opposite directions. The crystal in the PET scanner may obtain the photons as PET scanning data. In some embodiments, the PET scanner may obtain specific PET scanning data by setting one or more PET scanning parameters. For example, the PET scanning parameter may include a coincidence window width, an FOV of scanning, a scanning time, or the like, or any combination thereof.

In some embodiments, the plurality of PET scanners may have the same structure. For example, a PET ring inside the PET scanner 1 may have the same radius, the same type of detector elements, and/or the same number of detector rings as that of a PET ring inside the PET scanner 2. In some embodiments, the plurality of PET scanner may perform scanning on the target object based on one or more same scanning parameter(s). For example, the PET scanner 1 may have same scanning parameters as the PET scanner 2 (e.g., the coincidence window width, the FOV of scanning, the scanning time.).

In some embodiments, the plurality of PET scanners may have different structures. For example, a detector ring inside the PET scanner 1 configured to scan patient's head may have a smaller radius. A detector ring inside the PET scanner 2 configured to scan patient's body (e.g., heart, abdomen, or other positions) may have a larger radius. In some embodiments, the plurality of PET scanners may scan on the target object based on different scanning parameters. For example, the PET scanner 1 may have scanning parameters different from the PET scanner 2 (e.g., the coincidence window width, the FOV of scanning, the scanning time).

The CT scanner 210 may perform CT scanning on the target object and obtain CT scanning data. The CT scanner 210 may include a radioactive source and a detector. The radioactive source may emit radioactive rays toward the target object. In some embodiments, the radioactive source may include a α ray source, a β ray source, a γ ray source, an X-ray source, a neutron source, an electron source, etc. The rays emitted by the radioactive source may include a fan beam, a parallel beam, a cone beam, etc. In some embodiments, the CT scanner 210 may include a plurality of radioactive sources. The plurality of radioactive sources may be of the same type or different types.

The detector may receive rays (also referred to herein as radiation) traversing through the target object and convert the radiation into a signal that can be processed by a computing device. In some embodiments, the detector may convert the received radiation into visible light and further convert the visible light into an electrical signal. The electrical signal may be converted by an analog/digital converter and then transmitted to the computing device 120 for imaging. The detector may include an arc detector, a circular detector, a square detector, or the like, or any combination thereof. In some embodiments, the detector may be an arc detector. In some embodiments, the CT scanner may include a plurality of detectors. The plurality of detectors may be arranged along the axial direction to form a detector array. In some embodiments, the CT scanner may further include one or more sensors. The one or more sensors may be configured to monitor operating status of the radioactive source and the detector (operating parameters, e.g., a radioactive source voltage, radioactive source current, a temperature of the detector, a delay value when the detector converts scanning signal). The one or more sensors may include a temperature sensor, a voltage sensor, a current sensor, or the like.

In some embodiments, the CT scanner 210 may scan the target object according to the one or more CT scanning parameters set. For example, the CT scanning parameter may include a scanning time, a scanning rate, a radioactive source voltage, a radioactive source current, etc. For example, the CT scanner may scan the target object at a specific power and obtain corresponding CT scanning data by setting the radioactive source voltage and the radioactive source current.

The driving device 215 may be configured to drive the PET scanning component 205 and the CT scanner 210. In some embodiments, the PET scanning component 205 and/or the CT scanner 210 may be mounted on the driving device 215. In some embodiments, the PET scanning component 205 and/or the CT scanner 210 may be mounted on the drive device 215 by using a slide rail, a chain, a belt, a screw, a bolt, a snap joint, or the like.

In some embodiments, the driving device 215 may include a driver. The driver may be configured to drive the PET scanning component 205 and/or the CT scanner 210 attached to the driving device to move. The driver may be driven by electric power, hydraulic pressure, air pressure, or the like, or any combination thereof. In some embodiments, the driver may be or include a motor. The motor may drive the PET scanning component 205 and/or the CT scanner 210 to move using an electrical driving method. For example, the motor may include a low speed motor (e.g., a gear motor, a claw pole synchronous motor), a high speed motor, a constant speed motor, a variable speed motor (e.g., an electromagnetic variable-speed motor, a speed-switched reluctance motor, a DC speed motor), a linear motor, or the like, or any combination thereof.

The driving device 215 may respectively drive the PET scanning component 205 and/or the CT scanner 210 to move by the driver. The movement may include axial translation, circumferential rotation, tilting, swing, or the like, or any combination thereof. For example, the driving device 215 may respectively drive the PET scanner 1, the PET scanner 2, . . . , the PET scanner n to move along the axial direction to a designated location. As another example, the driving device 215 may drive the CT scanner 210 to perform axial translation and circumferential rotation simultaneously to achieve helical scanning of the target object. In some embodiments, the imaging apparatus 110 may include a plurality of driving devices 215. One or more of the PET scanner 1, the PET scanner 2, ... , the PET scanner n and/or the CT scanner 210 may be mounted on different driving devices. In some embodiments, at least one PET scanner of the PET scanning component 205 may be fixed. The driving device 215 may drive other PET scanners to move along the axial direction. For example, the PET scanner 1 may be fixed in the axial direction to scan the patient's head. The driving device 215 may drive one or more PET scanners (e.g., PET scanner 2, . . . , PET scanner n) to move along the axial direction to scan other parts of the patient, e.g., torso, limbs.

For persons having ordinary skills in the art, after understanding the basic principles of the imaging apparatus, the modules may be combined in various ways, or connected with other modules as sub-systems without departing from the principles. Various variations and modifications may be conducted under the teaching of the present disclosure. However, those variations and modifications are still within the scope of the present disclosure described above. For example, the imaging apparatus 110 may only include the PET scanning component 205 and a driving device 215. As another example, the imaging apparatus 110 may further include an MRI scanner for performing magnetic resonance imaging on the target object. As still another example, the imaging apparatus 110 may also include a user interface. A user may operate the PET scanning component 205, the CT scanner, and/or the driving device 215 through the user interface.

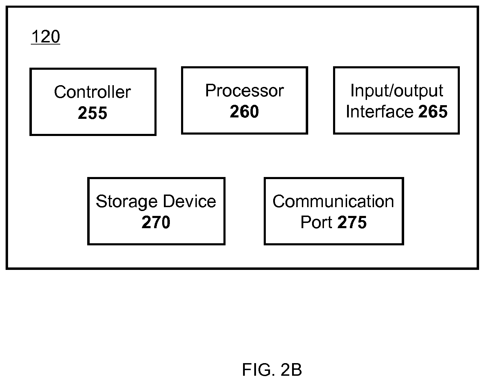

FIG. 2B is a schematic diagram of an exemplary computing device according to some embodiments of the present disclosure. The computing device 120 may include a controller 255, a processor 260, an input/output interface 265, a storage device 270, and a communication port 275. The controller 255 may be configured to make a decision and generate a control instruction. The controller 255 may receive request or command information inputted through the input/output interface 265, information inputted through the communication port 275, information generated by the processor 260, or information stored in the storage device 270. The controller 255 may make the decision and generate the control instruction based on the information. In some embodiments, the control instruction may be transmitted to the imaging apparatus 110 for setting scanning parameters and driving the scanner(s) to move. For example, the controller 255 may transmit a control instruction of moving the PET scanner to a location to the driving device 215 for driving the PET scanner to move to the corresponding location. In some embodiments, the control instruction may be transmitted to one or more components of the computing device 120. For example, the control instruction may be transmitted to the input/output interface 265 for reminding a user to perform inputting or other control operations.

In some embodiments, the controller 255 may include a control unit or a device in the computing device 120. For example, the controller 255 may include a microcontroller unit (MCU), a central processing unit (CPU), a programmable logic device (PLD), an application specific integrated circuits (ASIC), a single chip microcomputer (SCM), a system on a chip (SoC), or the like. As another example, the controller 255 may be a specially designed unit or device that has specific control functions.

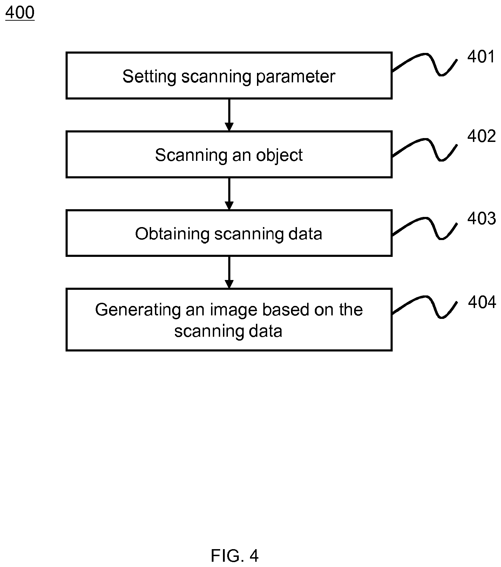

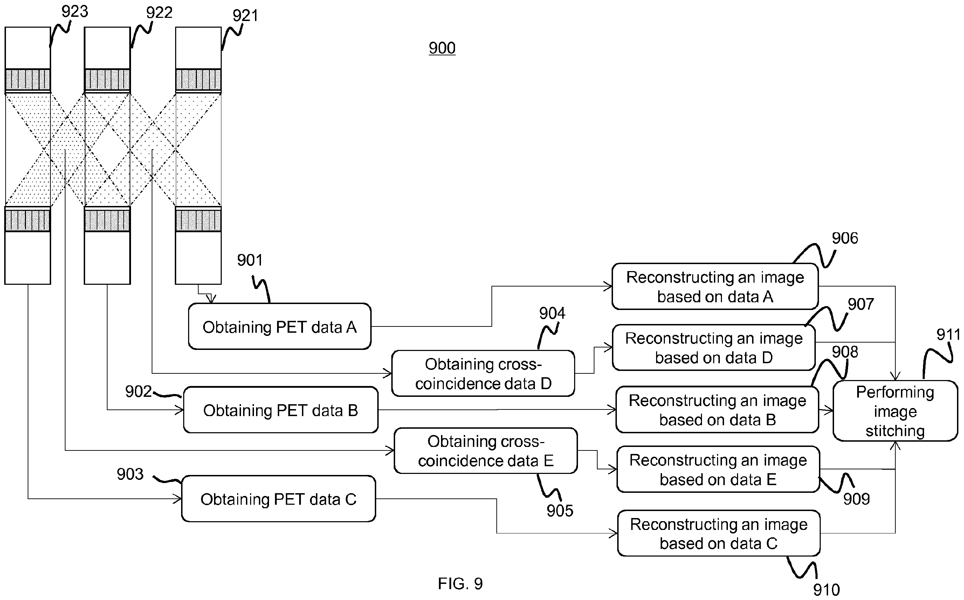

The processor 260 may be configured to process data. The processor 260 may obtain information from the imaging apparatus 110, the input/output interface 265, the storage device 270, or the communication port 275. In some embodiment, the processor 260 may process the obtained information through one or more processing techniques. The processing technique may include fitting, interpolation, discretization, analog-to-digital conversion, Z-transform, Fourier transform, low-pass filtering, edge denoising, feature extraction, image reconstruction, image enhancement, or the like, or any combination thereof. In some embodiments, the processor 260 may perform operations of reconstruction or generate a scanning image in processes described in FIG. 4 and/or FIG. 9.

The processor 260 may include a processing unit or a device, e.g., a central processing unit (CPU), a digital signal processor (DSP), a graphics processing unit (GPH), or the like. In some embodiments, the processor 260 may include a specially designed unit or a device that may have a specific function. For example, the processor 260 may be a processing unit or device that is designed according to a standard of Digital Imaging and Communication in Medicine (DICOM).

The input/output interface 265 may be configured to receive user input information or output information generated by the computing device 120. The information inputted through the input/output interface 265 may be in the form of number, text, image, audio, video, or the like. The input/output interface 265 may obtain information from the user by a handwriting operation, a mouse operation, a touch screen operation, a key operation, a voice control operation, a gesture operation, an eye operation, a voice operation, or the like. The information inputted through the input/output interface 265 may be stored in the storage device 270 or transmitted to the controller 255 or processor 260 for further processing. The computing device 120 may output a processing result through the input/output interface 265 or transmit a request for obtaining information to the user. In some embodiments, the information outputted through the input/output interface 265 may be in the form of number, text, audio, image, light, vibration, or the like, or any combination thereof.

In some embodiments, the input/output interface 265 may input or output information through a physical interface, for example, a touch screen, a microphone, a speaker, an LDE indicator, a button, a key, or the like. In some embodiments, the input/output interface 265 may input or output information, for example, virtual reality, holographic images, through a virtual interface. In some embodiments, the input/output interface 265 may include a LED display screen, a LED indicator, a speaker, a button, a key, or the like, or any combination thereof in the computing device 120.

The storage device 270 may perform a function of storing information for the computing device 120. The storage device 270 may store information in the form of text, number, audio, image, or the like. The storage device 270 may also store instructions or codes executed by the controller 255 and/or the processor 260. When the controller 255 and/or the processor 260 execute the codes, the computing device 120 may perform one or more functions of the computing device 120 described in the present disclosure. In some embodiments, the storage device 270 may include, but is not limited to, various types of storage devices e.g., a solid state disk, a mechanical hard disk, a universal serial bus (USB) flash memory, a secure digital (SD) memory card, an optical disk, a random-access memory (RAM), a read-only memory (ROM). In some embodiments, the storage device 270 may include a storage device of the system, an external storage device that may be connected to the system, a network storage device outside the system (e.g., storage on a cloud storage server).

The communication port 275 may construct a communication between the computing device 120 and the network or other external devices. The communication may include a wired communication and wireless communication. The wired communication may include using a transmission medium such as a wire, a cable, an optical cable, a waveguide, a nanomaterial. The wireless communication may include IEEE 802.11 series wireless local area network communication, IEEE 802.15 series wireless communication (e.g., Bluetooth, ZigBee), mobile communication (e.g., TDMA, CDMA, WCDMA, TD-SCDMA, TD-LTE, FDD-LTE), satellite communication, microwave communication, scattering communication, etc. In some embodiments, the communication port 275 may be a general communication port (e.g., RS485, RS232), or a specially designed communication port according to a particular standard. For example, the communication port 275 may be designed according to the standard of Digital Imaging and Communication in Medicine (DICOM).

For persons having ordinary skills in the art, after understanding the basic principles of the computing device, the modules may be combined in various ways, or connected with other modules as sub-systems without departing from the principles. Various variations and modifications may be conducted under the teaching of the present disclosure. However, those variations and modifications are still within the scope of the present disclosure described above. For example, the controller 255 and the processor 260 may be integrated into a system on a chip (SoC) for processing data and making a decision and generating a control instruction.

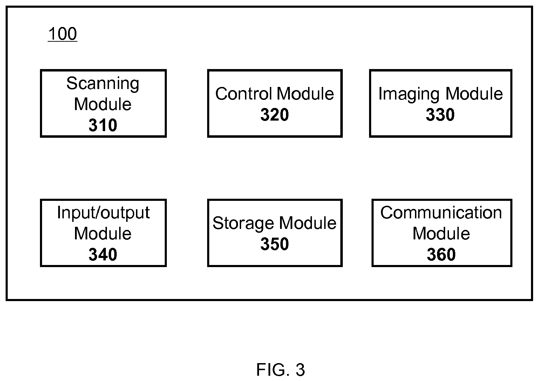

FIG. 3 is a schematic diagram of an exemplary imaging system according to some embodiments of the present disclosure. The imaging system 100 may include a scanning module 310, a control module 320, an imaging module 330, an input/output module 340, a storage module 350, and a communication module 360. The connection among different modules may be wired, wireless, or a combination thereof. Any of the modules may be local, remote, or a combination thereof. A corresponding relationship among the modules may be one to one or one to many. For example, the imaging system 100 may include a plurality of scanning modules 310 and a plurality of control modules 320. Each control module 320 may control a scanning module 310, respectively, to obtain corresponding information of a scanning object (e.g., the target object). As another example, the imaging system 100 may include a plurality of scanning modules 310 and a control module 320. The control module 320 may control the plurality of scanning modules 310 to obtain information of the scanning object.

The scanning module 310 may scan the target object and obtain corresponding scanning data. In some embodiments, the scanning module 310 may include one or more scanners. For example, the scanner may include a PET scanner, a CT scanner, an MRI scanner, a B scanner, a thermal tomographic scanner, or the like, or any combination thereof. The scanning data obtained by the scanning module 310 may include positron emission tomography (PET) scanning data, X-ray scanning data, magnetic resonance (MRI) scanning data, ultrasonic scanning data, thermal tomographic data, or the like, or any combination thereof. The scanning data may be derived from scanning one or more of objects such as an organism, an organ, tissue, a lesion. In some embodiments, the scanning module 310 may include one or more scanners and one or more driving devices. For example, the scanning module 310 may include at least two PET scanners, one CT scanner, and one driving device.

In some embodiments, the scanning data obtained by the scanning module 310 may be transmitted to the control module 320 for making a decision. In some embodiments, the scanning data obtained by the scanning 310 may be transmitted to the imaging module 330 for reconstructing an image. In some embodiments, the scanning data obtained by the scanning 310 may be stored in the storage module 350. In some embodiments, the scanning data obtained by the scanning 310 may be transmitted to a network or a database, a server, a terminal device that are connected to the network through the communication module 360. In some embodiments, the scanning module 310 may be implemented as one or more scanning equipment or scanning devices, for example, the imaging apparatus 110.

The control module 320 may provide decision information for the imaging system 100 and generate a corresponding control instruction. The control module 320 may receive a request or command information inputted through the input/output module 340, information inputted through the communication module 360, an image or a processing result generated by the imaging module 330, and information stored in the storage module 350. The control module 320 may make a decision and generate the control instruction based on the information. For example, the control module 320 may receive information inputted by a user through input/output module 340, and determine one or more PET scanning locations and generate a control instruction of moving corresponding PET scanners to the one or more PET scanning locations. In some embodiments, the control instruction may be transmitted to the scanning module 310 for setting scanning parameters and/or driving scanners to move. For example, the control module may transmit the control instruction of moving the PET scanner to a location to the scanning module 310 for driving the PET scanner to move to the location. In some embodiments, the control instruction may be transmitted to other modules of the imaging system 100. For example, the control instruction may be transmitted to the input/output module 340 for reminding the user to perform inputting or other control operations. In some embodiments, the control module 320 may be implemented as one or more control units, for example, the controller 255 of the computing system 120.

The imaging module 330 may process scanning data obtained by the imaging system 100 and generate an image. The scanning data may be obtained by the scanning module 310 performing a CT scanning or PET scanning on the target object, obtained from the storage module 350 or obtained through the communication module 360. In some embodiments, the imaging module 330 may preprocess the obtained scanning data. The pre-processing may include filtering, regularization, etc. In some embodiments, the imaging module 330 may reconstruct the image based on the scanning data. The image reconstruction operation may include interpolation, fitting, iteration, Fourier transform, convolution back projection, etc. In some embodiments, the imaging module 330 may perform post-processing on the reconstructed image. The post-processing may include image enhancement, image segmentation, image denoising, imaging geometric correction, edge feature extraction, image stitching, etc. In some embodiments, the imaging module 330 may be implemented as one or more processing units or devices, for example, the processor 260 of the computing device 120.

The input/output 340 may receive user input information or transmit information generated by the imaging system to a user. The information inputted through the input/output module 340 may be in the form of number, text, image, audio, image, light, vibration, etc. For example, the user may input one or more operation instructions through the input/output module 340. The operation instruction may include an instruction of setting scanning parameters for the scanning module 310, an image processing request, etc. The information inputted through the input/output module 340 may be stored in the storage module 350, or transmitted to the control module 320 or the imaging module 330 for further processing. The input/output module 340 may output information in one or more forms of light, text, audio, image, vibration, or the like. For example, the imaging system 100 may output the generated medical image through input/output module 340. In some embodiments, the input/output module 340 may be or include one or more physical units or devices, for example, a touch screen, a LED indicator, a speaker, a microphone, or the like. In some embodiments, the input/output module 340 may be integrated into a console of the imaging system 100. In some embodiments, the input/output 340 may be implemented as one or more input/output interfaces or devices, for example, the input/output interface 265 of the computing device 120.

The storage module 350 may store information obtained and/or generated by the imaging system 100. The information stored in the storage module 350 may include the scanning data obtained by the scanning module 310, the decision information and/or the control instruction generated by the control module 320, the processing result of the scanning data generated by the imaging module 330, information obtained through the input/output module 340, and information obtained through the communication module 360, etc. The storage module 350 may store information in the form of text, table, image, video, etc. In some embodiments, the storage module 350 may be a local storage, external storage, or storage (e.g., a cloud storage) connected to the imaging system through a communication module, etc. In some embodiments, the storage module 350 may implement as one or more storage devices, for example, the storage device 270 of the computing device 120.

The communication module 360 may construct a communication between the imaging system and the network or another external device. The communication may include a wired communication or wireless communication. The wired communication may include using a transmission medium such as a wire, a cable, an optical cable, a waveguide, a nanomaterial. The wireless communication may include IEEE 802.11 series wireless local area network communication, IEEE 802.15 series wireless communication (e.g., Bluetooth, ZigBee), mobile communication (e.g., TDMA, CDMA, WCDMA, TD-SCDMA), satellite communication, microwave communication, etc. In some embodiments, the communication module 360 may select different transmission modes based on the type of data to be transmitted or different types of networks.

The imaging system 100 may connect to the network through the communication module 360. The network may be a single network or a combination of multiple networks. In some embodiments, the network may include multiple network access points, for example, a wired or wireless access point, a base station, or a network switching point. Through an access point, the imaging 100 system may connect to the network and transmit or receive information through the network. In some embodiments, the imaging system 100 may interact with a server, a database, or a terminal device that are connected to the network through the communication module 360. In some embodiments, the communication module 360 may be implemented as one or more communication ports or devices, for example, the communication port 275 of the computing device 120.

FIG. 4 is a flowchart of an exemplary process for generating a scanning image according to some embodiments of the present disclosure. In 401, the control module 320 may set one or more scanning parameters. The scanning parameter may include one or more CT scanning parameters and/or one or more PET scanning parameters. The CT scanning parameter may include, but is not limited to, a scanning time, a scanning range, object location information, a scanning bed location, a CT scanner rotation speed, a scanning bed feed speed, a voltage and current intensity, signal acquisition frequency, etc. The PET scanning parameter may include, but is not limited to, a scanning time, an axial scanning range, object location information, a scanning bed location, a scanning bed feed speed, a sampling frequency, a window width, an axial FOV of scanning, etc. The scanning parameter may be a default value, a value set by a user through the input/output module 340, or a value adaptively adjusted by the imaging system 100. In some embodiments, the scanning parameter may be set according to the target object to be scanned. The object may include a human body, an animal, an abiotic object, etc. For example, the target object may include a limb, an organ, tissue, a tumor, or the like, or any combination thereof. For example, when the imaging system 100 performs PET scanning on the patient's head and abdomen, the imaging system 100 may select different PET scanning parameters such as different scanning times, different window widths, different scanning ranges, different scanning bed feed speeds. The control module 320 may generate a corresponding control instruction based on the scanning parameters.

In 402, the scanning module 310 may scan the target object. The scanning module 310 may receive the control instruction generated by the control module 320 and scan the target object. In some embodiments, one or more scanners (e.g., the PET scanning component 205, the CT scanner 210) may scan the object by emitting a beam toward the object. For example, the beam toward the target object may include a α-ray beam, a β-ray beam, a γ-ray beam, an X-ray beam, a neutron beam, an electron beam, a visible light beam, or the like. In some embodiments, the one or more scanners may scan the target object by receiving a beam from the target object. For example, the beam from the target object may include a γ photon beam, an infrared ray beam, or the like. The one or more scanners may scan the target object in one or more scanning modes. The scanning mode may include translation scanning, rotation scanning, helical scanning, swing-rotation scanning, etc.

In 403, the scanning module 310 may obtain the scanning data. The scanning module 310 may obtain the scanning data through one or more signal obtaining elements (e.g., the detector of the CT scanner, the detector ring or the detector element of the PET scanner). In some embodiments, the scanning module 310 may obtain the scanning data through one or more conversion processes. For example, the scanning module 310 may convert the obtained X-ray signal into a visible optical signal. As another example, the imaging system 100 may convert the visible optical signal into a current or voltage signal. As still another example, the scanning module 310 may further convert an analog signal into a digital signal and convert the digital signal into scanning data that the imaging module 330 can process.

In 404, the imaging module 330 may generate an image based on the scanning data. In some embodiments, the imaging module 330 may receive the scanning data obtained by the scanning module 310 and generate an image. The imaging module 330 may process the obtained scanning data through one or more processing techniques and generate the image. The processing technique may include pre-processing, image reconstruction, post-processing, etc. The pre-processing may include filtering, discretizing, and denoising the obtained scanning data. The imaging reconstruction may include Fourier transform, convolution back projection, iteration, interpolation, fitting, etc. The imaging system may select different image reconstruction algorithms for different scanning data. For example, for the PET scanning data, the imaging module 330 may reconstruct the image by using a Conjugate Gradient algorithm, a Maximum A Posteriori algorithm, an iteration algorithm, or the like. For the CT scanning data, the imaging module 330 may reconstruct the image by using a Filtering reconstruction algorithm, a Radon inversion algorithm, an image Hilbert transform, or the like. For the MRI scanning data, the imaging module 330 may reconstruct the image by using a Fourier transform method, an interpolation method, an iteration method, or the like.

The image may include a PET image, a CT image, an MRI image, etc. In some embodiments, the image may be any combination of the above-described images. For example, the imaging module 330 may reconstruct a PET/ CT image based on the PET scanning data and the CT scanning data. As another example, the imaging module may reconstruct a PET/MRI image based on the PET scanning data and the MRI scanning data. In some embodiments, the imaging module may process the reconstructed image through one or more post-processing techniques. The post-processing technique may include pseudo-color enhancement, edge regularization, segmentation based on region, image rendering, image distortion correction, artifact correction, etc. The pre-processing technique, the image reconstruction, and the post-processing technique may be implemented by one or more of linear algebra, calculus, numerical analysis, image processing, digital signal processing, or the like.

It should be noted that the above description of the process of generating the image based is provided for the purpose of illustration, and not intended to limit the scope of the present disclosure. For persons having ordinary skills in the art, the operations may be exchanged or combined in various ways. Various variations and modifications may be conducted under the teaching of the present disclosure. However, those variations and modifications may not depart from the scope of this disclosure. For example, the imaging system 100 may store or transmit the reconstructed image.

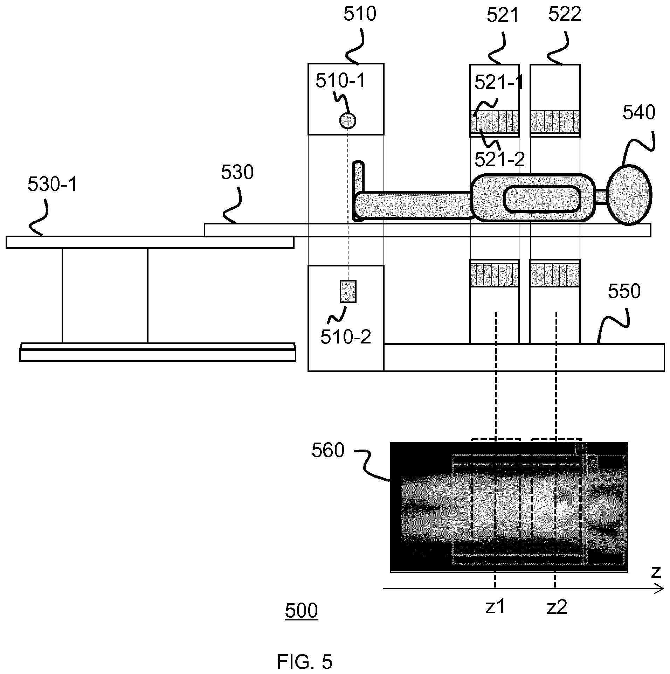

FIG. 5 is a block diagram of an exemplary scanning module according to some embodiments of the present disclosure. The scanning module 500 may include a CT scanner 510, a PET scanner 521, a PET scanner 522, a scanning bed 530, and a driving device 550.

The scanning bed 530 may support a target object (e.g., a patient 540 to be scanned). In some embodiments, a bed board of the scanning bed 530 may move along an axial direction (Z direction shown in FIG. 5). The control module 320 may control the movement of the bed board. During the scanning process, the control module 320 may perform a scanning on the target object by controlling the bed board of the scanning bed 530 to move along the Z direction and pass through one or more scanning areas of the scanners. A moving speed of the bed board of the scanning bed 530 may be constant or vary. In some embodiments, the moving speed of the bed board of the scanning bed 530 may relate to a parameter, e.g., a scanning time, a size of the scanning area, or the like. In some embodiments, the moving speed of the bed board of the scanning bed 530 may be a default value, a value set by a user through the input/output module 340, or an adaptive value adjusted by the imaging system 100. For example, when a user does not set the moving speed of the bed board of the scanning bed 530, the control module 320 may determine that the moving speed of the bed board of the scanning bed 530 is a default value. When the imaging system 100 receives the moving speed of the bed board of the scanning bed 530 set by a user, the control module 320 may determine that the moving speed of the bed board of the scanning bed 530 is the value set by the user. In some embodiments, the bed board of the scanning bed 530 may be fixed. During the scanning, the control module 320 may perform the scanning on the target object by controlling the CT scanner 510, the PET scanner 521, and/or the PET scanner 522 to move along the axial direction and pass through the target object.

In some embodiments, the control module 320 may perform a one-dimension coding on the scanning bed 530 along the axial direction (e.g., the Z direction shown in FIG. 5) to generate a bed-code value for each location of the scanning bed 530. In some embodiments, a bed-code value at any location on the scanning bed 530 may be determined by setting the bed-code value of the starting point location of the scanning bed 530 (e.g., one end 530-1 of the scanning bed 530) as zero. The bed-code value of one location may correspond to a relative distance between the location and the starting point location. In some embodiments, the bed-code value of the location may be equal to the relative distance between the location and the starting point location. The bed-code values of the scanning bed 530 may be stored in the storage device 270 in the form of numbers, texts, tables, vectors, or the like. In some embodiments, the control module 320 may perform a two-dimension or three-dimensional coding on the scanning bed 530.

In some embodiments, the scanning bed 530 may include one or more mechanical devices, for example, a lifting link, a transmission component, or the like. The mechanical device may be used to lift and/or turn the bed body of the scanning bed 530. The lifting of the bed body of the scanning bed 530 may include an entirety lifting, a slope lifting, etc., of the bed body. The lifting and/or turning of the bed body may be controlled by the control module 320, manually adjusted by a user, or adaptively adjusted by the imaging system 100 according to a need of scanning.

The CT scanner 510 may perform a CT scanning on the target object and obtain CT scanning data. The CT scanner 510 may include a radioactive source 510-1 and a detector 510-2. In some embodiments, the radiation source 510-1 may include an X-ray tube. During the scanning, the radioactive source 510-1 may emit radioactive rays to the patient 540. Due to the differences in different tissues, different parts of the patient may have different levels of absorption to incident rays. So the rays transmitted from various parts of the patient may have different intensities. The detector 510-2 may receive transmitted rays with different intensities to generate the CT scanning data. In some embodiments, the transmitted rays may be associated with one or more scanning parameters of the CT scanner 510. The scanning parameter may include a scanning time, a scanning range, a rotational speed of the CT scanner, a voltage and a current value of a radioactive source, or the like. In some embodiments, the CT scanner 510 may scan a patient in one or more scanning modes. The scanning mode may include translational scanning, rotational scanning, helical scanning, swing-rotational scanning, or the like, along the axial direction. In some embodiments, the translation, rotation, and/or swing of the CT scanner 510 along the axial direction may be driven by the driving device 550.

The imaging module 330 may reconstruct a CT image based on the CT scanning data obtained by the CT scanner 510. The algorithm used to reconstruct the CT image may include a parallel beam projection algorithm, a parallel beam back-projection filter reconstruction algorithm, a fan-shaped beam back-projection filter reconstruction algorithm, an iterative reconstruction algorithm, or the like. For example, the parallel beam projection algorithm may include a direct Fourier transform reconstruction algorithm, a filtered back-projection reconstruction algorithm, a Radon inversion algorithm, or the like. The parallel beam back projection filtering algorithm may include a Hilbert transform of a unary function, a Hilbert transform on a finite interval, a Hilbert transform of an image, or the like. The imaging module 330 may obtain scanning data of a whole body or a local part of a patient obtained by the CT scanner 510 and reconstruct a CT scanning image of the whole body or the local part of the patient.

In some embodiments, the imaging module 330 may perform one or more processing on the reconstructed CT image. The processing may include image enhancement, image segmentation, artifact removal, image coding, or the like, or any combination thereof. In some embodiments, the imaging module 330 may code the CT image based on the bed-code value(s) of the scanning bed 530 to generate a CT scout image (e.g., a CT scout image 560 shown in FIG. 5). A portion of the CT scout image may correspond to a location of the scanning bed 530. In some embodiments, the code value of a CT scout image corresponding to a same location of the scanning bed 530 may be the same as the bed-code value of the location. For example, locations with code values as z1 and z2 in the CT scout image may be the same as locations with bed-code values as z1 and z2 in the corresponding scanning bed 530. The coding may include a one-dimensional coding, a two-dimensional coding, or a three-dimensional coding. In some embodiments, the scanned image in the CT scout image may have a certain accuracy. The control module 320 may determine one or more regions of interest (ROI) by the CT scout image, for example, an organ in the patient, an abnormal tissue, or the like. In some embodiments, the CT scout image may be used to determine an area to be scanned (e.g., an organ, an area of abnormal tissue) and then determine one or more PET scanning locations. The control module 320 may obtain the one or more PET scanning locations and control one or more PET scanners to move to one or more corresponding PET scanning locations.

The PET scanner 521 may scan the target object and generate PET scanning data. The PET scanner 521 may include a plurality of PET detector rings (e.g., a PET detector ring 521-1, a PET detector ring 521-2, as shown in FIG. 5). Before PET scanning, an agent labeled with one or more radionuclides (e.g., F18, C11) may be injected into the body of the patient 540. The radionuclide may produce positrons during a decay process in the body of the patient. The positrons may annihilate after combining with electrons at an adjacent position and generate a γ photon pair emitting in opposite directions. One or more detector rings of the PET scanner 521 may obtain the γ photon pair as PET scanning data. In some embodiments, the PET scanner 521 may obtain specific PET scanning data by setting one or more PET scanning parameters. The PET scanning parameter may include a window width, an FOV of scanning, a scanning time, or the like. For example, the γ photon pair in opposite directions may be received by two PET scanners and be determined as cross-coincidence PET scanning data (also referred to herein as cross-coincidence data) by setting one or more PET scanning parameters. In some embodiments, the cross-coincidence data may be received by two adjacent PET scanners. In some embodiments, the cross-coincidence PET scanning data may be sent to the imaging module 330 for processing. The imaging module 330 may process the cross-coincidence data by one or more processing methods. For example, when two adjacent PET scanners are far away from each other, the imaging module 330 may correct the cross-coincidence data by applying a weighting coefficient or a weight matrix to the cross-coincidence data. The weight coefficient or the weight matrix may relate to a distance between the two PET scanners.

In some embodiments, the imaging module 330 may correct the PET scanning data by one or more correction processing. The correction processing may include radionuclide decay correction, tissue decay correction, random coincidence correction, scattering correction, dead time correction, or the like, or any combination thereof. In some embodiments, the imaging module 330 may reconstruct an image using the corrected PET scanning data. A reconstruction algorithm may include a filtered back-projection reconstruction algorithm, an iterative reconstruction algorithm, or the like. For example, the iterative reconstruction algorithm may include a Maximum Likelihood-Expectation Maximum, a Conjugate gradient, a Maximum A Posteriori, or the like. In some embodiments, the imaging system 100 may scan a particular part of a patient through the PET scanner 521 and reconstruct a PET image of the particular part.

In some embodiments, the PET scanner 522 may be implemented in a similar manner as the PET scanner 521. In some embodiments, the structure of PET scanner 522 may be the same as the structure of the PET scanner 521. For example, detector rings inside the PET scanner 522 and the PET scanner 521 may have the same diameter, detection element type, and quantity. In some embodiments, the PET scanner 522 and the PET scanner 521 may scan the target object based on one or more same scanning parameters. For example, the PET scanner 522 may have same scanning parameters (e.g., a scanning time, a window width, an FOV of scanning) as that of the PET scanner 521.

In some embodiments, the structure of the PET scanner 522 may be different from the structure of the PET scanner 521. For example, compared with the PET scanner 521, the PET scanner 522 may have detector rings with a different diameter or a different quantity and/or its detector rings may be composed of different shapes, different numbers, or different types of detection elements. In some embodiments, the scanning parameter of the PET scanner 522 may be different from the scanning parameter of the PET scanner 521. For example, compared with the PET scanner 521, the PET scanner 522 may have a different count rate, scanning time, scanning mode (e.g., two-dimensional scanning, three-dimensional scanning), window width, bed, or the like. In some embodiments, a PET data storage way, an image reconstruction algorithm based on the PET scanning data, etc., generated by the PET scanner 522 may be different from that of the PET scanner 521.

The driving device 550 may drive the PET scanner 521, the PET scanner 522, and the CT scanner 510 to move. The movement may include axial translation, circumferential rotation, tilting swing, or the like, or any combination thereof. For example, the driving device 550 may independently drive the PET scanner 521 and/or the PET scanner 522 to respectively move along the axial direction to a designated location, and scan the target object. As another example, the driving device 550 may drive the CT scanner 510 to perform an axial movement and a circumferential rotation to achieve helical scanning of the target object. In some embodiments, the PET scanner 521, the PET scanner 522, and/or the CT scanner 510 may be mounted on the driving device 550. In some embodiments, the scanning module 500 may include a plurality of driving devices. One or more of the PET scanner 521, the PET scanner 522, and/or the CT scanner 510 may be mounted on different driving devices. In some embodiments, at least one of the PET scanner 521 and the PET scanner 522 may be fixed. The driving device 550 may drive an unfixed PET scanner to move along the axial direction. For example, the PET scanner 522 may be fixed along the axial direction to scan the head of a patient; the driving device 550 may drive the PET scanner 521 to move along the axial direction to scan other part (e.g., the trunk, limbs) of a patient.

In some embodiments, the driving device 550 may drive the PET scanners 521 and 522 to move to a corresponding PET scanning location and scan the target object at the PET scanning location. In some embodiments, a user may determine the PET scanning location. The user may determine one or more PET scanning locations on the CT scout image by a touch screen operation, a button or a key operation, a gesture operation, a voice operation, an eye operation, or the like (e.g., the PET scanning location may correspond to one or more organs in the CT scout image 560). For example, a user may draw one or more identifiers on the CT scout image through the input/output module 340 by clicking and dragging a mouse to determine a PET scanning location. The identifier may include a dot, a circle, a box, or a pattern with an arbitrary shape. The control module 320 may receive the user operation and determine the PET scanning location based on the received user operation. In some embodiments, the control module 320 may obtain one or more parameters inputted by a user to determine a PET scanning location. The parameter may include an image coding value, a bed-code value, an organ name, or the like, or any combination thereof. For example, the control module 320 may obtain two bed-code values inputted by a user and determine the locations corresponding to the two bed-code values as two PET scanning locations. In some embodiments, the PET scanning location may be determined by the control module 320. For example, if the head and the heart are two target parts, the control module 320 may automatically identify the heart and the head of the human in the scanning process based on the CT scout image and characteristic information (e.g., a boundary, a CT value) of the target part.

In some embodiments, the control module 320 may determine the PET scanning location(s). For example, by comparing the CT scout image and a CT scanning image of normal human tissue, the control module 320 may determine that a location corresponding to one or more abnormal tissues is the PET scanning location. In some embodiments, both the control module 320 and a user may determine the PET scanning location. For example, the control module 320 may select one or more PET scanning locations (e.g., by comparing the CT scout image and a CT scanning image of a normal human tissue, the control module 320 may make an identifier on one or more positions with abnormal tissue of the CT scout image). The user may adjust one or more PET scanning locations selected by the control module 320 through the input/output module 340 (e.g., modify the location of the identifier). The control module 320 may receive the operation of the user and determine a PET scanning location based on the received operation of the user. After determining the PET scanning location, the control module 320 may control the driving device 550 to drive one or more PET scanners to move to one or more corresponding PET scanning locations and scan the target object in one or more areas corresponding to the one or more PET scanning locations. For example, the control module 320 may control the driving device 550 to drive the PET scanner 521, and the PET scanner 522 to PET scanning locations corresponding to z1 and z2, respectively, and scan the chest and abdomen of the patient 540.

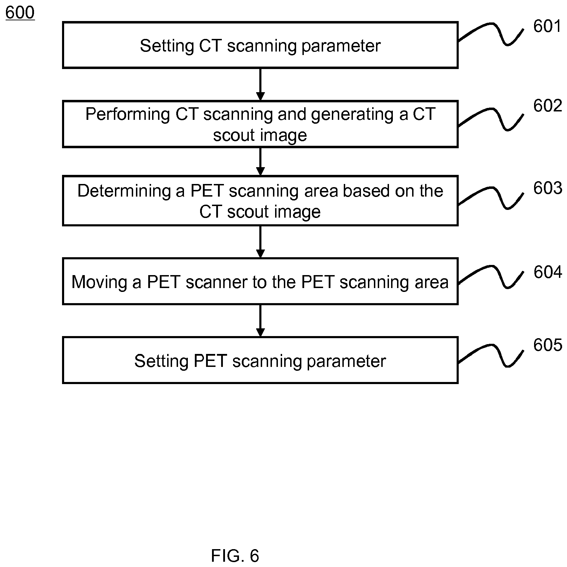

FIG. 6 is a flowchart illustrating an exemplary process for setting one or more PET scanning parameters according to some embodiments of the present disclosure. In some embodiments, steps 401 and 402 in FIG. 4 may be performed according to process 600. As shown in FIG. 6, the control module 320 may set a CT scanning parameter in 601. The CT scanning parameter may include voltage, current, beam shape, scanning time, scanning mode (e.g., helical scanning, translational scanning, and rotational scanning), scanning speed, sampling frequency, or the like, or any combination thereof. The CT scanning parameter may be a default value, a value set by a user through the input/output module 340, or an adaptive value adjusted by the PET system. For example, the control module 320 may detect a current status of the system before setting the CT scanning parameter (e.g., a stability of the voltage and current of the radioactive source in the CT scanner detected by a sensor, a delay value when a detector converts a scanning signal). The CT scanning parameter may be adaptively adjusted according to the current status of the system. In some embodiments, step 601 may further include setting a CT data storage parameter (e.g., a sequence of filling a sinogram data matrix, a dimension of a data matrix), a CT image reconstruction parameter (e.g., a parallel beam back projection filtering reconstruction parameter, a fan-shaped beam back projection filtering reconstruction parameter, an iterative reconstruction parameter), a CT image processing parameter (e.g., an image enhancement parameter, an image sharpening parameter, an image denoising parameter), by the control module 320. In some embodiments, the scanning parameter may be set by a user through the input/output module 340. The control module 320 may generate a corresponding control instruction based on the scanning parameter.

In 602, the scanning module 310 may perform a CT scanning and generate a CT scout image. The scanning module 310 may perform the CT scanning on the target object using the CT scanner based on the CT scanning parameter set in 601. The scanning may include helical scanning, rotational scanning, translational scanning, swing-rotational scanning, or the like, or any combination thereof. In some embodiments, during scanning, the CT scanner may perform a rotational scanning, and the scanning bed 530 may translate along the axial direction. In some embodiments, during scanning, the CT scanner may perform a helical scanning or a rotational-translational scanning, and the scanning bed 530 may be fixed. In some embodiments, the scanning module 310 may drive the CT scanner to scan the target object through the driving device 215 and obtain CT scanning data.

The imaging module 330 may reconstruct a CT image based on the scanning data obtained by the scanning module 310 after completing CT scanning. The reconstruction algorithm used to reconstruct the CT image may include a parallel beam projection algorithm, a parallel beam back-projection filter reconstruction algorithm, a fan-shaped beam back-projection filter reconstruction algorithm, an iterative reconstruction algorithm, or the like, or any combination thereof. For example, the parallel beam projection algorithm may include a direct Fourier transform reconstruction algorithm, a filtered back-projection reconstruction algorithm, a Radon inversion algorithm, or the like. The parallel beam back projection filtering algorithm may include a Hilbert transform of unary functions, a Hilbert transform on a finite interval, a Hilbert transform of the image, or the like. In some embodiments, the imaging module 330 may perform one or more post-processing on the reconstructed CT image. The post-processing may include image enhancement, image segmentation, artifact removal, image coding, or the like, or any combination thereof. In some embodiments, the imaging module 330 may code the CT image based on the code values of the portions of the scanning bed 530, and generate a CT scout image with code values (e.g., the exemplary CT scout image 560 shown in FIG. 5). The code may include a one-dimensional code, a two-dimensional code, or a three-dimensional code. A portion of the CT scout image may correspond to a location on the scanning bed 530 by coding. In some embodiments, a code value in the CT scout image corresponding to the same location of the scanning bed 530 may be the same as a bed-code value of the location.

In 603, the control module 320 may determine one or more PET scanning locations based on the CT scout image. In some embodiments, a user may determine the PET scanning location(s). The user may determine one or more PET scanning locations on the CT scout image through the input/output module 340 by a touch screen operation, a button or a key operation, a gesture operation, a voice operation, an eye operation, or the like (e.g., the PET scanning location(s) may correspond to one or more positions of one or more organs in the CT scanning image 560). For example, a user may draw one or more identifiers on the CT scout image by clicking and dragging a mouse to determine the PET scanning location. The identifier may include a dot, a circle, a box, or a pattern with an arbitrary shape. The control module 320 may receive the user operation and determine the PET scanning location based on the received user operation. In some embodiments, the control module 320 may obtain one or more parameters inputted by the user to determine the PET scanning location. The parameter may include an image coding value, a bed-code value, an organ name, or the like, or any combination thereof. For example, the control module 320 may obtain two bed-code values inputted by the user and determine locations corresponding to the two bed-code values as two PET scanning locations.

In some embodiments, the PET scanning location may be determined by the control module 320. For example, by comparing the CT scout image and a CT scanning image of a normal human tissue, the control module 320 may determine one or more locations corresponding to one or more abnormal tissues as the PET scanning location(s). In some embodiments, both the control module 320 and a user may determine the PET scanning location(s). For example, the control module 320 may select one or more PET scanning locations (e.g., by comparing the CT scout image and a CT scanning image of a normal human tissue, the control module 320 may make an identifier on one or more positions with abnormal tissue of the CT scout image). The user may adjust one or more PET scanning locations selected by the control module 320 through the input/output module 340 (e.g., modify the location of the identifier). The control module 320 may receive the adjusted operation and determine the PET scanning location(s) based on the received adjustment operation of the user. At 604, the scanning module 310 may move the PET scanner(s) to the PET scanning locations. The scanning module 310 may obtain the PET scanning location(s) determined by the control module 320 at 603 and move one or more PET scanners to one or more corresponding locations. In some embodiments, the scanning module 310 may determine the location by obtaining an image coding value corresponding to the identifier in the CT scout image. In some embodiments, if the PET scanning location is determined by one or more bed-code values at 603, the scanning module 310 may move the PET scanner to a corresponding location based on the bed-code value. In some embodiments, the scanning module 310 may move the PET scanner to a designated location by one or more driving devices (e.g., the driving device 215). In some embodiments, the scanning module 310 may perform scanning on a patient by moving the bed board of the scanning bed to pass through scanning area of the PET scanner.

At 605, the control module 320 may set one or more PET scanning parameters. The PET scanning parameter may include a coincidence window width, an FOV of scanning, scanning time, or the like, or any combination thereof. The PET scanning parameter may be a system default value, a value set by a user through the input/output interface 265, or an adaptive value adjusted by the system. For example, before setting the CT scanning parameter, the control module 320 may obtain information, e.g., diameter, width, shape, quantity, type of the detector ring of the PET scanner, and/or a position to be scanned. Based on the information, the control module 320 may adaptively adjust the PET scanning parameter(s). In some embodiments, the operation at 605 may further include setting a PET data storage parameter, a PET image reconstruction parameter, a PET image processing parameter, or the like, or any combination thereof.

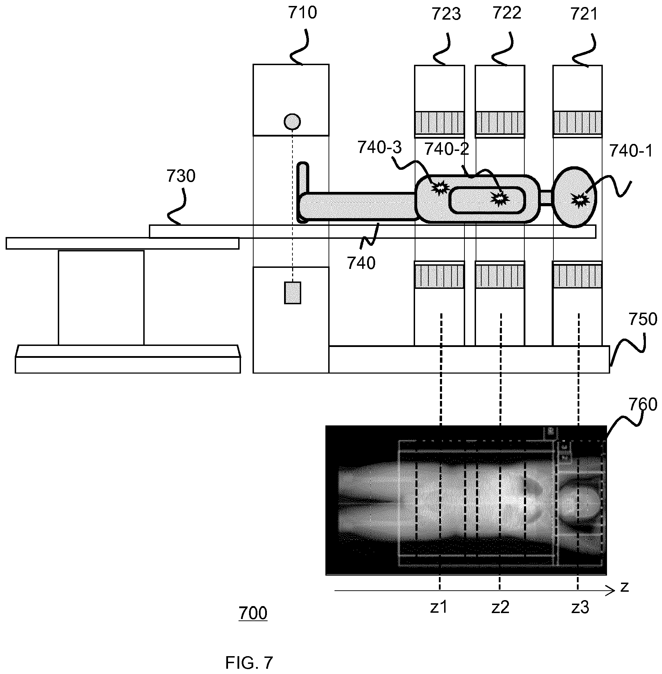

FIG. 7 is a block diagram of an exemplary scanning module 700 according to some embodiments of the present disclosure. The scanning module 700 may include a CT scanner 710, three PET scanners 721-723, a scanning bed 730, and a driving device 750. The scanning bed 730 may support a patient 740. The driving device 750 may drive the CT scanner 710 to perform CT scanning on the patient 740 and obtain scanning data. During the CT scanning, the bed board of the scanning bed 730 may translate along the axial direction, and the driving device 750 may drive the CT scanner 710 to rotate around the axis direction to realize the scanning of the patient 740. After the scanning, the scanning data may be transmitted to the imaging module 330 to generate an image. The imaging module 330 may generate a CT scout image (e.g., an exemplary CT scout image 760 shown in FIG. 7) based on the CT scanning data obtained by the CT scanner 710.

The control module 320 may determine one or more PET scanning locations based on the CT scout image. In some embodiments, the PET scanning location may be determined by a user. For example, the user may drag a scout identifier on the CT scout image to an area where the PET scanning is to be performed (e.g., head, liver, and abdomen of the patient) by a touch screen operation. The scout identifier may include a point, a vertical line, a cross cursor, a circle, a box, or the like, or any combination thereof. The control module 320 may receive the user operation and determine one or more PET scanning locations based on the received user operation. In some embodiments, the control module 320 may obtain one or more parameters inputted by a user to determine the PET scanning locations. The parameter may include an image coding value, a bed-code value, an organ name, or the like, or any combination thereof. For example, the control module 320 may obtain two bed-code values inputted by the user and determine locations corresponding to the two bed-code values as two PET scanning locations.

In some embodiments, the control module 320 may determine the PET scanning location(s). For example, by comparing the CT scout image and a CT scanning image of the normal human tissue, the control module 320 may determine one or more locations corresponding to one or more abnormal tissues as the PET scanning location(s). In some embodiments, the PET scanning location may be determined by both of the control module 320 and a user. For example, the control module 320 may select one or more PET scanning locations (e.g., by comparing the CT scout image and a CT scanning image of a normal human tissue, the control module 320 may make an identifier in one or more positions with abnormal tissue according to the CT scout image). The user may adjust one or more PET scanning locations selected by the control module 320 through the input/output module 340 (e.g., modify the location of the identifier). The control module 320 may receive the adjusted operation and determine the PET scanning location based on the received adjustment operation of the user.

After determining the PET scanning location(s), the driving device 750 may drive one or more PET scanners respectively to move to one or more corresponding locations, and perform PET scanning. For example, the control module 320 may determine the code values of the positions to be scanned are z1, z2, and z3, according to the CT scout image 760. The control module 320 may control the driving device 750 to drive the PET scanner 721, the PET scanner 722, and the PET scanner 723 to move to corresponding locations along the axial direction, respectively, based on the code values z1, z2, and z3 corresponding to the location of the scanning bed 730 In some embodiments, the PET scanner 721, the PET scanner 722, and the PET scanner723 may perform PET scanning simultaneously and obtain PET scanning data of scanning areas corresponding to the scanning locations. For example, the PET scanner 721, the PET scanner 722, and the PET scanner 723 may scan a head region, a thoracic region, and an abdomen region of the patient simultaneously, and obtain PET scanning data. The PET scanning data may be transmitted to the imaging module 330 for reconstructing one or more PET scanning images of the scanning areas. In some embodiments, the PET scanners 721-723 may have the same or different axial or radial FOVs of scanning. For example, the PET scanner 721 may include two detector rings, and the PET scanners 722 and 723 may include three or more detector rings. The PET scanner 722 and the PET scanner 723 may have a wider axial FOV of scanning as compared with that of the PET scanner 721. In some embodiments, at least one of the PET scanners 721, 722, and 723 may be fixed. The driving device 750 may drive the one or more unfixed PET scanners to move along the axial direction. For example, the PET scanner 721 may be fixed along the axial direction to scan the head of the patient, the PET scanners 722 and 723 may move along the axial direction to scan other positions of the patient, e.g., trunk, limbs.

For persons having ordinary skills in the art, after understanding the basic principles of the scanning module, units may be combined in various ways, or connected with other units as sub-units. Various variations and modifications may be made. However, those variations and modifications may not depart from the scope of this disclosure. For example, the scanning module 700 may include any number (e.g., four, five) of PET scanners. As another example, the multiple PET scanning areas may be interrelated. Merely by way of example, after the control module 320 receives an identifier draw by a user and determine a corresponding location (e.g., determine the liver position as a PET scanning location by the identifier), the control module 320 may then relate to multiple PET scanning locations (locations corresponding to e.g., the head, the kidney) based on the quantity of the PET scanners and perform PET scanning on the multiple locations.

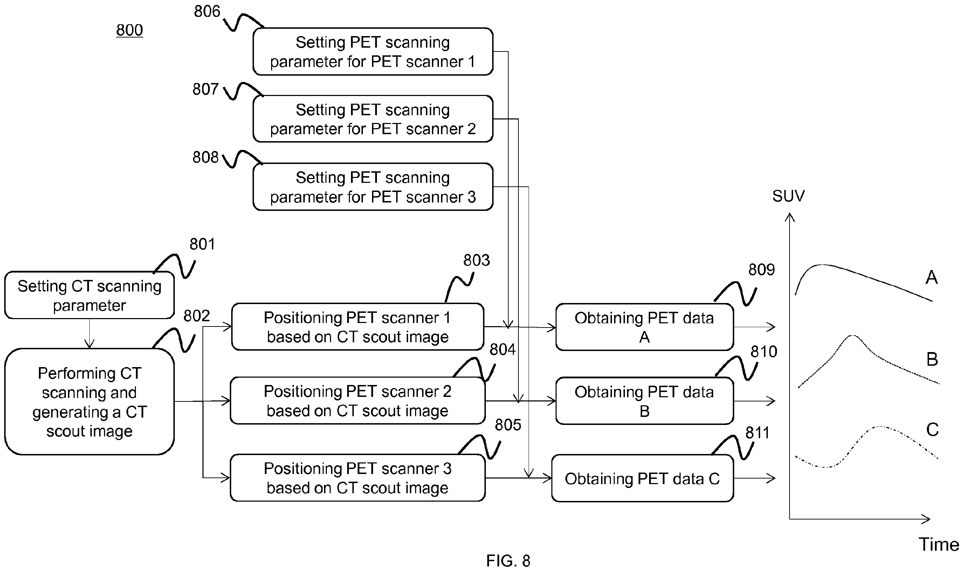

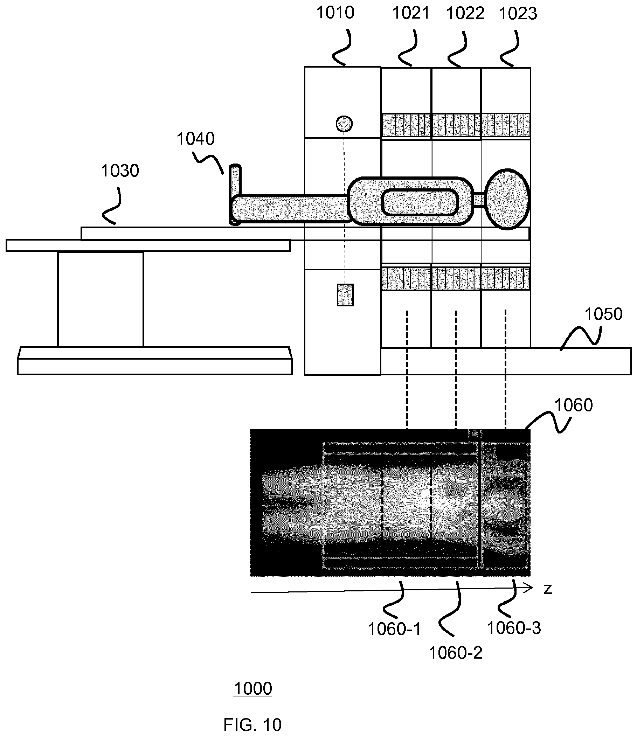

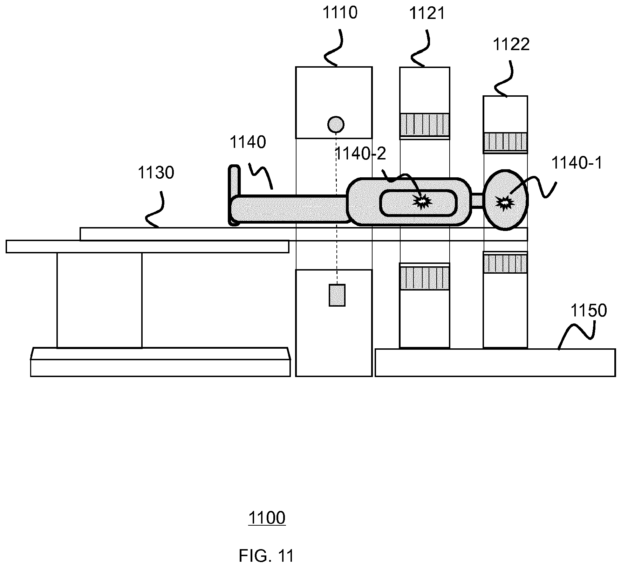

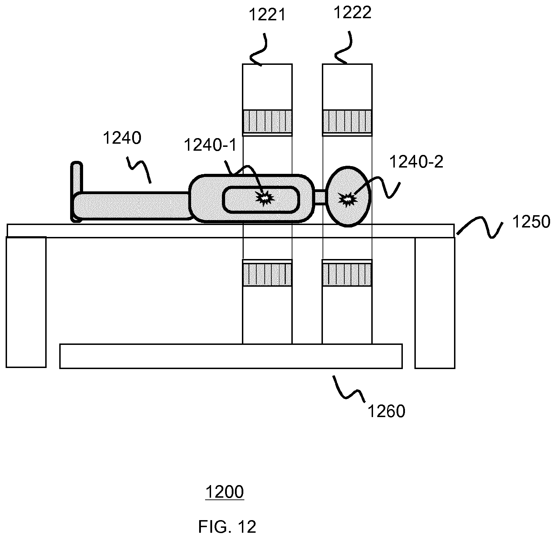

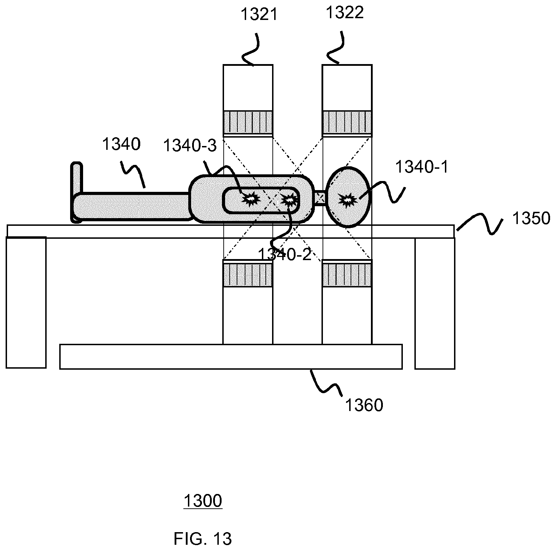

FIG. 8 is a flowchart illustrating an exemplary process for obtaining PET scanning data according to some embodiments of the present disclosure. In some embodiments, process 800 may be implemented by a scanning module as described in other parts of the present disclosure, for example, the scanning module 310 described in FIG. 3, the scanning module 500 described in FIG. 5, the scanning module 700 described in FIG. 7, the scanning module 1000 described in FIG. 10, the scanning module 1100 described in FIG. 11, the scanning module 1200 described in FIG. 12, or the scanning module 1300 described in FIG. 13. At 801, the control module 320 may set a CT scanning parameter. The CT scanning parameter may include a voltage, a current, a beam shape, scanning time, a scanning mode, a scanning speed, a sampling frequency, or the like, or any combination thereof. The CT scanning parameter may be a default value, a value set by a user through the input/output interface 265, or an adaptive value adjusted by the system. In some embodiments, the scanning parameter may be set via the input/output module 320 by a user. In some embodiments, the control module 320 may generate corresponding control instructions based on the CT scanning parameter.

In 802, the scanning module 310 may perform CT scanning based on the obtained CT scanning parameter. The scanning module 310 may perform imaging on a target object and obtain scanning data based on the control instructions generated by the control module 320 in 801. After completing scanning, the imaging module 330 may obtain the scanning data for reconstructing a CT image. A reconstruction algorithm used to reconstruct the CT image may include a parallel beam projection algorithm, a parallel beam back-projection filter reconstruction algorithm, a fan-shaped beam back projection filter reconstruction algorithm, an iterative reconstruction algorithm, or the like. In some embodiments, the imaging module 330 may code the reconstructed CT image. In some embodiments, the imaging module 330 may code based on the correspondence relationship between a bed-code value of the scanning bed and the CT image and generate a CT scout image. In some embodiments, the operations included in 801 and 802 may be the same as or similar to those included in 601 and 602 in process 600, respectively.

In 803, the control module 320 may determine a scanning location for the PET scanner 1 based on the CT scout image. In some embodiments, a user may determine the scanning location of the PET scanner 1. The control module 320 may send the CT scout image to the user through the input/output module 340 or one or more external terminal devices connected to the communication module 360. The user may determine one or more PET scanning locations on the CT scout image by a touch screen operation, a button or a key operation, a gesture operation, a voice operation, an eye operation, or the like. For example, the user may draw one or more boxes on the CT scout image by opening, closing, moving, fingers on the touch screen to determine the PET scanning locations. The control module 320 may receive the user input and determine the PET scanning location based on the received user input. In some embodiments, the control module 320 may position a PET scanner through acquiring one or more parameters. The parameter(s) may include an image coding values, a bed-code value, an organ to be scanned, or the like. For example, the control module 320 may receive two bed-code values inputted by the user through the input/output module 340. The control module 320 may then determine locations corresponding to the two bed-code values as the scanning locations of the PET scanner 1.

In some embodiments, the control module 320 may determine the scanning location of the PET scanner 1. For example, by comparing the CT scout image and a CT scanning image of the normal human tissue, the control module 320 may determine one or more locations corresponding to one or more abnormal tissues as the PET scanning location(s). In some embodiments, the PET scanning location may be determined by both of the control module 320 and the user. For example, the control module 320 may select one or more PET scanning locations (e.g., by comparing the CT scout image and a CT scanning image of a normal human tissue, the control module 320 may make an identifier in one or more positions with abnormal tissue of the CT scout image). The user may adjust one or more PET scanning locations selected by the control module 320 through the input/output module 340 (e.g., modify the location of the identifier). The control module 320 may receive the adjusted operation and determine the PET scanning location of the PET scanner 1 based on the received adjustment operation of the user.

**The** control module 320 may control the PET scanner 1 to move to a corresponding location after determining the PET scanning location. In some embodiments, the control module 320 may control the PET scanner 1 to move to a position (e.g., head position) of a patient. In some embodiments, the control module 320 may scan a corresponding position of the patient through controlling the bed board of the scanning bed translate and pass through the scanning area of the PET scanner 1. In some embodiments, the operations included in 803 may be the same as or similar to those included in 603 and 604 in process 600.

In 804 and 805, the control module 320 may control the PET scanner 2 and the PET scanner 3 in the scanning module 310 to move to designated locations, respectively. In some embodiments, the operations included in 804 and 805 may be the same as or similar to those included in 803. For example, the PET scanner 2 may be moved and positioned to the heart position of the patient; the PET scanner 3 may be moved and positioned to the abdomen position of the patient. In some embodiments, the operations included in 803, 804, and 805 may be performed simultaneously (e.g., the control module 320 may control the PET scanner 1, the PET scanner 2, and the PET scanner 3 to move to designated locations simultaneously based on the CT scout image).

In 806, the control module 320 may set one or more scanning parameters of the PET scanner 1. The PET scanning parameter(s) may include a scanning time, a sampling frequency, a window width, a window location, or the like, or any combination thereof. The PET scanning parameter may be a default value, a value set by a user through the input/output module 340, or an adaptive value adjusted by the system. For example, before setting the PET scanning parameter, the control module 320 may determine information of the PET scanner and/or information of a body part to be scanned. The information of the PET scanner may include, e.g., a diameter, a width of the detector ring and/or a shape, quantity, a type of the detector element. The information of the body part to be scanned may include a volume of the body part to be scanned, a diameter of a maximum circumscribed circle, a tissue type, or the like, or any combination thereof. The control module 320 may adaptively adjust the PET scanning parameter based on the information. In some embodiments, the operations included in 806 may be the same as or similar to the operations included in 605 in process 600.

In 807 and 808, the control module 320 may respectively set scanning parameters of the PET scanner 2 and the PET scanner 3. In some embodiments, the operations included in 807 and 808 may be the same as or similar to the operations included in 605 in process 600. In some embodiments, the scanning parameters respectively set for the PET scanner 1, the PET scanner 2, and the PET scanner 3 in 806, 807, and 808 may be the same or different. For example, the scanning parameter of the PET scanner 1 may be set separately according to the needs for head scanning. The scanning parameters of the PET scanner 2 and the PET scanner 3 may be set in the same way according to the needs for body scanning.

In 809, the scanning module 310 may obtain PET data A. The PET scanner 1 may scan a body part of a patient based on the scanning parameter set in 806 and obtain corresponding scanning data. For example, the PET scanner 1 may obtain scanning data of brain statically. In some embodiments, the PET scanner 1 may scan a body part of the patient in a scanning mode, e.g., translation scanning, rotation scanning, swing scanning, or the like, or any combination thereof, and collect corresponding scanning data. In some embodiments, the PET scanning data may be obtained by acquiring γ photon pairs generated in a scanning area in real time. The form of the PET scanning data may include a curve, a table, a text, or the like, or any combination thereof. In some embodiments, the PET scanner 1 may obtain scanning data at multiple time points in real time. The scanning data of the multiple time points may be represented in the form of a dynamic curve. For example, scanning data at multiple time points may be represented as PET curve A. The PET curve A may be a curve of Standardized Uptake Value (SUV) over time. The SUV may be related to metabolism of an agent in the scanning area. In some embodiments, the SUV may be used to determine a level of metabolism of tissue in the scanning area. For example, the level of SUV in different positions of the same organ may be used to determine the metabolic level of the different positions. In some embodiments, the SUV of a malignant tumor tissue is higher than that of a benign tumor.

In 810 and 811, the scanning module 310 may obtain PET data B and PET data C of corresponding scanning areas. For example, the scanning module 310 may obtain dynamic cardiac PET data B and dynamic abdominal PET data C. In some embodiments, the operations included in 810 and 811 may be the same as or similar to those included in 809. In some embodiments, the operations included in 809, 810, and 812 may be performed simultaneously. For example, the scanning module 310 may obtain dynamic data of the brain, heart, and abdomen simultaneously based on the scanning parameters set in 806, 807, and 808, respectively.

It should be noted that the above description of the process for obtaining PET scanning data is provided for the purpose of illustration, and not intended to limit the scope of the present disclosure. For persons having ordinary skills in the art, steps may be combined in various ways or switched with other steps. Various variations and modifications may be conducted after understanding the process. However, those variations and modifications may not depart from the scope of this disclosure. For example, the PET scanner 1, the PET scanner 2, and the PET scanner 3 may have different structures and/or scan the brain, heart and abdomen positions based on different scanning parameters and obtain the dynamic scanning data of corresponding positions simultaneously.

FIG. 9 is a flowchart illustrating an exemplary process for processing the obtained PET scanning data according to some embodiments of the present disclosure. In some embodiments, the operations included in process 900 may be implemented by a scanning module as described in other parts of the present disclosure. For example, the scanning module 310 described in FIG. 3, the scanning module 500 described in FIG. 5, the scanning module 700 described in FIG. 7, the scanning module 1000 described in FIG. 10, the scanning module 1100 described in FIG. 11, the scanning module 1200 described in FIG. 12, the scanning module 1300 described in FIG. 13, or the like. In 901, the imaging system 100 may obtain PET data A (e.g., dynamic brain data) of a corresponding area through the PET scanner 921. In some embodiments, the operations included in 901 may be the same as or similar to those included in 809 in process 800.

In 902 and 903, the scanning module 310 may obtain PET data B (e.g., dynamic cardiac data) and PET data C (e.g., dynamic abdominal data). In some embodiments, the operations included in 902 and 903 may be the same as or similar to those included in 901. In some embodiments, the operations included in 901, 902, and 903 may be performed simultaneously. For example, the scanning module 310 may obtain dynamic data of the brain, heart, and abdomen simultaneously.

In 904, the scanning module 310 may obtain cross-coincidence data D. In some embodiments, cross-coincidence data D may be generated based on γ photon pairs emitted in an area between the PET scanner 921 and the PET scanner 922. The γ photon pairs may be obtained by the PET scanner 921 and the PET scanner 922, respectively. In some embodiments, cross-coincidence data D may be generated by both of the PET scanner 921 and the PET scanner 922. In some embodiments, cross-coincidence data D may be transmitted to the imaging module 330. The imaging module 330 may process the cross-coincidence data D by one or more processing methods. For example, the imaging module 330 may filter the obtained cross-coincidence data D to remove γ photon pairs with emission angles greater than a certain threshold. As another example, the imaging module 330 may multiply the cross-coincidence data D by a weighting coefficient that is between 0 and 1. The weighting coefficient may be related to a distance between the PET scanner 921 and the PET scanner 922.

In 905, the scanning module 310 may obtain cross-coincidence data E. In some embodiments, the operations included in 905 may be the same as or similar to those included in 904. In some embodiments, the operations in 904 and the operations in 905 may be performed simultaneously.

In 906, the imaging module 330 may reconstruct a PET image (e.g., a PET scanning image of brain) of a corresponding area based on the PET data A (e.g., the dynamic brain data). The imaging module 330 may image the brain by one or more reconstruction techniques. The reconstruction technique may include a filter back projection reconstruction algorithm, an iterative reconstruction algorithm, or the like. For example, the iterative reconstruction algorithm may include the Maximum Likelihood-Expectation Maximum, Conjugate gradient, Maximum A Posteriori, or the like, or any combination thereof. In some embodiments, the imaging module 330 may process the reconstructed image by one or more post-processing techniques. The post-processing technique may include image enhancement, image geometry processing, image denoising, image feature extraction, or the like, or any combination thereof. For example, the image enhancement may include histogram enhancement, pseudo-color enhancement, grayscale window enhancement, or the like, or any combination thereof. The image geometry processing may include image scaling, distortion correction, skew correction, area calculation, or the like, or any combination thereof. The image denoising may include filtering, edge regularization, or the like, or any combination thereof. The image feature extraction may include the extraction of image edge features, the extraction of image texture features, the extraction of image shape features, the extraction of image contour features, or the like, or any combination thereof.

In 907-910, the imaging module 330 may reconstruct images of corresponding positions (e.g., positions between brain and heart, heart, positions between heart and abdomen, abdomen) based on the PET scanning data D, the PET scanning data B, the PET scanning data E, and the PET scanning data C, respectively. In some embodiments, the image reconstruction algorithms used in 906-910 may be the same or different. In some embodiments, the imaging module 330 may select a reconstruction algorithm based on the part to be imaged. For example, the imaging module 330 may reconstruct images of brain and heart using different reconstruction algorithms.

In 911, the imaging module 330 may stitch one or more reconstructed images in 906-910. The stitching processing may include image registration, image stitching, image post-processing, or the like, or any combination thereof. The image registration may perform many operations on the image, for example, geometric correction, projection transformation, uniform scale operation, or the like, or any combination thereof. The technique of image registration may include a phase correlation technique, a time domain based technique, or the like, or any combination thereof. In some embodiments, the imaging module 330 may register multiple images by drawing a positioning line. The positioning line may include location information and registration relationship among a plurality of images. In some embodiments, the positioning line may be automatically drawn by the imaging module 330 in a default manner, manually drawn by a user, or automatically drawn by the imaging module 330 according to an actual image. In some embodiments, the imaging module 330 may draw the positioning line based on code values of the respective images. The image stitching may include pixel-level stitching, feature-level stitching, decision-level stitching, or the like, or any combination thereof. The image post-processing may include contrast adjustment, brightness adjustment, soft processing, smoothing, or the like, or any combination thereof. In some embodiments, the user may equalize differences in the stitching location by selecting one or more post-processing methods based on a stitched image.

In some embodiments, the PET scanners 921-923 may scan from the head of the patient to the abdomen of the patient simultaneously, and reconstruct scanning images of respective areas based on the scanning data A-E. The scanning images of respective areas may be stitched into a full PET scanning image that may include the position of head of the patient to the abdomen of the patient.

FIG. 10 is a block diagram of an exemplary scanning module according to some embodiments of the present disclosure. The scanning module 1000 may include a CT scanner 1010, three PET scanners 1021-1023, a scanning bed 1030, and a driving device 1050. The scanning bed 1030 may support a patient 1040. During the scanning process, the bed board of the scanning bed 1030 may translate along the axial direction. In some embodiments, the driving device 1050 may drive the CT scanner 1010 to scan the patient 1040 and acquire the scanning data. After completion of the CT scanning, the imaging module 330 may generate a CT scout image (e.g., an exemplary CT scout image 1060 shown in FIG. 10) based on the scanning data acquired by the CT scanner 1010.

The control module 320 may determine scanning locations (also referred to herein as PET scanning locations) of the PET scanner 1021, the PET scanner 1022, and the PET scanner 1023 based on the CT scout image. In some embodiments, the determination of the PET scanning locations by the control module 320 may be found in other parts of the present disclosure, as shown in the description of FIG. 5, FIG. 6, FIG. 7, or FIG. 8. In some embodiments, the scanning areas of the PET scanner 1021, the PET scanner 1022, and the PET scanner 1023 may be 1060-1, 1060-2, and 1060-3, respectively. In some embodiments, the PET scanner 1021, the PET scanner 1022, and the PET scanner 1023 may have the same or different fields of view (FOV) of scanning along an axial direction or a radial direction. Accordingly, the sizes of the scanning areas 1060-1, 1060-2, and 1060-3 may be the same or different. In some embodiments, the scanning areas 1060-1, 1060-2, and 1060-3 may be tightly connected to each other.

The driving device 1050 may drive the PET scanner 1021, the PET scanner 1022, and the PET scanner 1023 to the corresponding locations (i.e., the PET scanning locations) and scan the corresponding areas (i.e., the scanning areas 1060-1, 1060-2, and 1060-3), respectively. In some embodiments, the PET scanner 1021, the PET scanner 1022, and the PET scanner 1023 may scan the areas simultaneously and acquire PET scanning data dynamically. The imaging module 330 may generate PET scanning images of the scanning areas 1060-1, 1060-2, and 1060-3 based on the scanning data acquired by the PET scanner 1021-1023, respectively. The imaging module 330 may stitch the PET images of the scanning areas into an image of a complete scanning area.

It should be noted that the above description of the scanning module 1000 is provided for the purpose of illustration, and not intended to limit the scope of the present disclosure. For persons having ordinary skills in the art, various variations and modifications may be conducted on the configuration of the imaging apparatus under the teaching of the present disclosure. However, those variations and modifications may not depart the scope of this disclosure. In some embodiments, the scanning module 1000 may include any number of PET scanners. The PET scanners may scan a plurality of interconnected areas and acquire scanning data. The imaging module 330 may reconstruct PET scanning images of the respective areas based on the scanning data. In some embodiments, the imaging module 330 may stitch the scanned images of the respective areas into an image with a large FOV.

FIG. 11 is a block diagram of an exemplary scanning module according to some embodiments of the present disclosure. The scanning module 1100 may include a CT scanner 1110, two PET scanners 1121 and 1122, a scanning bed 1130, and a driving device 1150. The scanning bed 1130 may support a patient 1140. During the scanning process, the bed board of the scanning bed 1030 may translate along the axial direction. The driving device 1150 may drive the CT scanner 1110 to scan the patient 1140 and generate a CT scout image. The control module 320 may control the driving device 1150 based on the CT scout image. The driving device 1150 may drive the PET scanner 1121 and the PET scanner 1122 to move to designated locations (e.g., locations corresponding to heart 1140-2 and the heart 1140-1 of the patient 1140), and perform scan imaging.

**The** PET scanner 1121 may be different from the PET scanner 1122. In some embodiments, as compared with the PET scanner 1122, the PET scanner 1121 may have detector rings with different diameters and/or different widths, and/or have detector elements with different shapes, different numbers, and/or different types. In some embodiments, as compared with the PET scanner 1122, the PET scanner 1121 may have detector rings with different diameters. For example, the PET scanner 1121 may include one or more detector rings with a larger diameter for scanning the heart 1140-2 of the patient 1040. The PET scanner 1121 may include one or more detector rings with a smaller diameter for scanning the head 1140-1 of the patient 1040. In some embodiments, the imaging system 100 may respectively set different scanning parameters, data storage methods, and/or reconstruction methods for the PET scanner 1121 and the PET scanner 1122. In some embodiments, the PET scanner 1121 and the PET scanner 1122 may scan the areas simultaneously and acquire PET scanning data dynamically. In some embodiments, the imaging module 330 may reconstruct a PET scanning image of the scanning area corresponding to the scanning location based on the scanning data. In some embodiments, the PET scanners 1121 and 1122 may have the same or different axial or radial FOV of scanning. In some embodiments, at least one of the PET scanner 1121 and 1122 may be fixed. The driving device 1150 may drive an unfixed PET scanner to move along an axial direction. For example, the PET scanner 1122 may be fixed in the axial direction to scan the head 1140-1 of the patient 1140; the driving device 1150 may drive the PET scanner 1121 to move along an axial direction to scan other part (e.g., the trunk, limbs) of the patient 1140.

It should be noted that the above description of the scanning module 1100 is provided for the purpose of illustration, and not intended to limit the scope of the present disclosure. For persons having ordinary skills in the art, various variations and modifications may be conducted on the configuration of the imaging apparatus under the teaching of the present disclosure. However, those variations and modifications may not depart the scope of this disclosure. In some embodiments, the scanning module 310 may further include one or more specifically designed PET scanners for imaging a particular part, e.g., a PET scanner used for imaging limbs, neck, or the like, of the patient 1140.

FIG. 12 is a block diagram of an exemplary scanning module according to some embodiments of the present disclosure. The scanning module 1200 may include two PET scanners 1221 and 1222, a scanning bed 1250, and a driving unit 1260. The scanning bed 1250 may support the patient 1240. During the scanning process, the bed board of the scanning bed 1250 may be fixed. The driving unit 1260 may drive the PET scanner 1221 and the PET scanner 1222 to move along an axial direction to a designated PET scanning location for imaging. In some embodiments, the PET scanners 1221 and 1222 may have the same or different axial or radial FOV of scanning. In some embodiments, at least one of the PET scanner 1221 and 1222 may be fixed. The driving unit 1260 may drive an unfixed PET scanner to move along an axial direction. For example, the PET scanner 1222 may be fixed along an axial direction to scan the head of the patient 1240; the driving unit 1260 may drive the PET scanner 1221 to move along an axial direction to scan other part (e.g., the trunk, limbs) of the patient 1140.

In some embodiments, the PET scanning location may be determined by a user. For example, when a patient requires a PET scanning for a part of his body, the doctor may determine the scanning location based on the patient's disease and his/her body size. In some embodiments, the doctor may drive the PET scanner 1221 and the PET scanner 1222 to a corresponding location by controlling the driving unit 1260. For example, the doctor may estimate general locations of head 1240-2 and liver 1240-1 and determine bed-code values corresponding to locations of the positions of the patient. The control module 320 may control the driving unit 1260 according to the bed-code values inputted by a doctor. The driving unit 1260 may move the PET scanner 1221 and the PET scanner 1222 to locations corresponding to the positions 1240-1 and 1240-2, respectively, for PET scanning. As another example, the doctor may input a height of the patient 1240. The control module 320 may determine a distance between the head and/or the liver of the patient and his/her foot based on the height to further determine the bed-code values of the PET scanning locations. In some embodiments, the distance may be an average, a median, or the like, based on big data statistics. The distance may be obtained through a network, a database or a server connected to a network. The control module 320 may control the driving unit 1260 to move the PET scanner 1221 and the PET scanner 1222 to corresponding scanning locations based on the bed-code values. In some embodiments, the PET scanner 1221 and the PET scanner 1222 may scan areas corresponding to the locations simultaneously and acquire PET scanning data dynamically. In some embodiments, the imaging module 330 may reconstruct a PET scanning image of the scanning areas corresponding to the scanning locations based on the scanning data.

In some embodiments, the scanning module 1200 further includes an image sensor, for example, a camera, a webcam, or the like. The image sensor may be mounted on over a patient for acquiring an image of the patient. In some embodiments, the image may be used to determine the height, proportions of body parts, and other information of the patient. The control module 320 may determine a portion of a body part to be scanned. The control module 320 may also control the driving unit 1260 to drive the PET scanner 1221 and the PET scanner 1222 to corresponding locations based on the information.

FIG. 13 is a block diagram of an exemplary scanning module according to some embodiments of the present disclosure. The scanning module 1300 may include two PET scanners 1321 and 1322, a scanning bed 1350, and a driving device 1360. The scanning bed 1250 may support a patient 1240. During the scanning process, the bed board of the scanning bed 1250 may be fixed. The control module 320 may control the driving device 1360 to drive the PET scanner 1321 and the PET scanner 1322 to move along an axial direction to designated PET scanning locations for imaging. In some embodiments, the determination of PET scanning locations by the control module 320 may be found in other parts of the present disclosure, as shown in the description of FIG.12. In some embodiments, the PET scanners 1321 and 1322 may have the same or different axial or radial FOV of scanning. In some embodiments, at least one of the PET scanner 1321 and 1322 may be fixed. The driving device 1360 may drive an unfixed PET scanner to move along an axial direction. For example, the PET scanner 1322 may be fixed along an axial direction to scan the head 1340-1 of the patient 1340; the driving device 1360 may drive the PET scanner 1321 to move along an axial direction to scan other part (e.g., the trunk, limbs) of the patient 1340.

As shown in FIG. 13, the PET scanner 1321 and the PET scanner 1322 may scan kidney 1340-3 and the head 1340-1, respectively. The imaging module 330 may generate PET images of the positions based on the scanning data obtained by the PET scanner 1321 and the PET scanner 1322. In some embodiments, the scanning module 310 may acquire scanning data of the heart 1340-2. The scanning data may be acquired by both of the PET scanner 1321 and the PET scanner 1322. In some embodiments, the imaging module 330 may correct cross-coincidence data for the heart 1340-2. For example, the imaging module 330 may select the acquired cross-coincidence data to remove γ photon pairs that have an emission angle greater than a threshold. As another example, the imaging module 330 may multiply the acquired cross-coincidence data by a weighting coefficient that is between 0 and 1. The weighting coefficient may be related to the distance between the PET scanner 1321 and the PET scanner 1322. The imaging module 330 may reconstruct a PET image of the heart based on the corrected cross-coincidence data. In some embodiments, the PET scanner 1121 and the PET scanner 1122 may scan the above three areas simultaneously and obtain PET scanning data dynamically. In some embodiments, the imaging module 330 may reconstruct a full PET scanning image of the area based on the scanning data.

Having thus described the basic concepts, it may be rather apparent to those skilled in the art after reading this detailed disclosure that the foregoing detailed disclosure is intended to be presented by way of example only and is not limiting. Various alterations, improvements, and modifications may occur and are intended to those skilled in the art, though not expressly stated herein. These alterations, improvements, and modifications are intended to be suggested by this disclosure and are within the scope of the exemplary embodiments of this disclosure.

Moreover, certain terminology has been used to describe embodiments of the present disclosure. For example, the terms "one embodiment," "an embodiment," and/or "some embodiments" mean that a particular feature, structure or characteristic described in connection with the embodiment is included in at least one embodiment of the present disclosure. Therefore, it is emphasized and should be appreciated that two or more references to "an embodiment" or "one embodiment" or "an alternative embodiment" in various portions of this specification are not necessarily all referring to the same embodiment. Furthermore, the particular features, structures or characteristics may be combined as suitable in one or more embodiments of the present disclosure.

Further, it will be appreciated by one skilled in the art, aspects of the present disclosure may be illustrated and described herein in any of some patentable classes or context including any new and useful process, machine, manufacture, or composition of matter, or any new and useful improvement thereof. Accordingly, aspects of the present disclosure may be implemented entirely hardware, entirely software (including firmware, resident software, micro-code, etc.) or combining software and hardware implementation that may all generally be referred to herein as a "block," "module," "engine," "unit," "component," or "system." Furthermore, aspects of the present disclosure may take the form of a computer program product embodied in one or more computer readable media having computer readable program code embodied thereon.

Computer program code for carrying out operations for aspects of the present disclosure may be written in any combination of one or more programming languages, including an object-oriented programming language such as Java, Scala, Smalltalk, Eiffel, JADE, Emerald, C++, C#, VB. NET, Python or the like, conventional procedural programming languages, such as the "C" programming language, Visual Basic, Fortran 2003, Perl, COBOL 2002, PHP, ABAP, dynamic programming languages such as Python, Ruby and Groovy, or other programming languages. The program code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider) or in a cloud computing environment or offered as a service such as a Software as a Service (SaaS).

Furthermore, the recited order of processing elements or sequences, or the use of numbers, letters, or other designations, therefore, is not intended to limit the claimed processes and methods to any order except as may be specified in the claims. Although the above disclosure discusses through various examples what is currently considered to be a variety of useful embodiments of the disclosure, it is to be understood that such detail is solely for that purpose and that the appended claims are not limited to the disclosed embodiments, but, on the contrary, are intended to cover modifications and equivalent arrangements that are within the scope of the disclosed embodiments. For example, although the implementation of various components described above may be embodied in a hardware device, it may also be implemented as a software only solution-e.g., an installation on an existing server or mobile device.

**Similarly,** it should be appreciated that in the foregoing description of embodiments of the present disclosure, various features are sometimes grouped together in a single embodiment, figure, or description thereof for the purpose of streamlining the disclosure aiding in the understanding of one or more of the various inventive embodiments. This method of disclosure, however, is not to be interpreted as reflecting an intention that the claimed subject matter requires more features than are expressly recited in each claim. Rather, inventive embodiments lie in less than all features of a single foregoing disclosed embodiment.

In some embodiments, the numbers expressing quantities or properties used to describe and claim certain embodiments of the application are to be understood as being modified in some instances by the term "about," "approximate," or "substantially." For example, "about," "approximate," or "substantially" may indicate ±20% variation of the value it describes, unless otherwise stated. Accordingly, in some embodiments, the numerical parameters outlined in the written description and attached claims are approximations that may vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the application are approximations, the numerical values outlined in the specific examples are reported as precisely as practicable.

In closing, it is to be understood that the embodiments of the application disclosed herein are illustrative of the principles of the embodiments of the application. Other modifications that may be employed may be within the scope of the application. Thus, by way of example, but not of limitation, alternative configurations of the embodiments of the application may be utilized in accordance with the teachings herein. Accordingly, embodiments of the present application are not limited to that precisely as shown and described.

## Claims

1. A system for medical imaging comprising:
an image sensor configured to generate image data, wherein the image sensor is a camera;
an imaging apparatus (110) including:
a first PET scanner (205), a second PET scanner (205), and a driving device (215); and
a computing device (120) including:
a controller (255) configured to determine a first scanning location and a second scanning location based on the generated image data by the image sensor, and
a processor (260), wherein,
the driving device (215) is configured to drive the first PET scanner (205) and
the second PET scanner (205) to move to the first scanning location and the second scanning location, respectively,
the first PET scanner (205) and the second PET scanner (205) are configured to obtain first scanning data and second scanning data, respectively, and
the processor (260) is configured to generate a first image of a first scanning area corresponding to the first scanning location and a second image of a second scanning area corresponding to the second scanning location.

2. The system of claim 1, wherein the first PET scanner (205) includes a first PET detector ring and the second PET scanner (205) includes a second PET detector ring.

3. The system of claim 1, wherein
the first PET scanner (205) and the second PET scanner (205) are mounted on the driving device (215), and
the driving device (215) is configured to drive
the first PET scanner (205) and the second PET scanner (205) to move along an axial direction.

4. The system of claim 1, further comprising a CT scanner configured to obtain CT scanning data, wherein the processor (260) is further configured to generate a scout image based on the CT scanning data.

5. The system of claim 4, wherein
the scout image includes a first identifier and a second identifier, and
the controller (255) is configured to determine the first scanning location and the second scanning location by obtaining a location of the first identifier and a location of the second identifier.

6. The system of claim 1, wherein
the first PET scanner (205) is configured ot obtain the first scanning data at one or more first time points,
the second PET scanner (205) is configured to obtain the second scanning data at one or more second time points,
the processor (260) is configured to perform dynamic imaging on the first scanning area based on the first scanning data at the one or more first time points, and
the processor (260) is configured to perform dynamic imaging on the second scanning area based on the second scanning data at the one or more second time points.

7. The system of claim 6, wherein the processor (260) is configured to determine metabolism of an agent in the first scanning area and the second scanning area based on the dynamic imaging, and the metabolism changes over time.

8. The system of claim 1, wherein the processor (260) is configured to generate a third image of a third scanning area based on scanning data obtained by the first PET scanner (205) and the second PET scanner (205), the third scanning area being between the first scanning area and the second scanning area.

9. The system of claim 8, wherein to generate the third image of the third scanning area based on the scanning data obtained by the first PET scanner (205) and the second PET scanner (205), the processor (260) is further configured to:
obtain third scanning data through both the first PET scanner (205) and the second PET scanner (205); and
generate the third image of the third scanning area based on the third scanning data.

10. The system of claim 8, wherein the processor (260) is configured to generate a full image by stitching images of the first scanning area, the second scanning area, and the third scanning area that are generated based on the scanning data obtained by the first PET scanner (205) and the second PET scanner (205).

11. The system of claim 1, wherein,
the first PET scanner (205) is configured to obtain a first scanning parameter and to scan the first scanning area according to the first scanning parameter; and
the second PET scanner (205) is configured to obtain a second scanning parameter and to scan the second scanning area according to the second scanning parameter, the first scanning parameter being different from the second scanning parameter.

12. A system for medical imaging comprising:
an image sensor configured to generate image data, wherein the image sensor is a camera;
an imaging apparatus (110) including:
a first PET scanner (205),
a second PET scanner (205), and
a driving device (215); and
a computing device (120) including:
a controller (255) configured to determine a first scanning location and a second scanning location based on the generated image data by the image sensor, and
a processor (260), wherein:
the first PET scanner (205) is mounted in the first scanning location,
the driving device (215) is configured to drive the second PET scanner (205) to move to the second scanning location,
the first PET scanner (205) and the second PET scanner (205) are respectively configured to obtain first scanning data and second scanning data, and
the processor (260) is configured to generate a first image of a first scanning area corresponding to the first scanning location and a second image of a second scanning area corresponding to the second scanning location.

13. A method for medical imaging using the system of claim 1 or claim 12 comprising:
obtaining a first PET scanning parameter and a second PET scanning parameter;
determining a first scanning location and a second scanning location based on generated image data by an image sensor, wherein the image sensor is a camera;
generating first scanning data by scanning a first scanning area corresponding to the first scanning location according to the first PET scanning parameter;
generating second scanning data by scanning a second scanning area corresponding to the second scanning location according to the second PET scanning parameter;
generating a first image of the first scanning area based on the first scanning data; and
generating a second image of the second scanning area based on the second scanning data.

14. A non-transitory computer-readable medium storing instructions adapted to perform the method of claim 13.

## Patentansprüche

1. System für medizinische Bildgebung, umfassend:
einen Bildsensor, der konfiguriert ist, um Bilddaten zu erzeugen, wobei es sich bei dem Bildsensor um eine Kamera handelt;
eine bildgebende Einrichtung (110), mit:
einem ersten PET-Scanner (205), einem zweiten PET-Scanner (205), und einer Antriebsvorrichtung (215); und
eine Rechenvorrichtung (120), mit:
einer Steuereinheit (255), die konfiguriert ist, um auf Basis der erzeugten Bilddaten vom Bildsensor einen ersten Scanort und einen zweiten Scanort zu bestimmen, und
einem Prozessor (260), wobei
die Antriebsvorrichtung (215) konfiguriert ist, um den ersten PET-Scanner (205) und
den zweiten PET-Scanner (205) so anzutreiben, dass diese sich zum ersten Scanort bzw. dem zweiten Scanort bewegen,
der erste PET-Scanner (205) und der zweite PET-Scanner (205) konfiguriert sind, um erste Scandaten bzw. zweite Scandaten zu erhalten, und
der Prozessor (260) konfiguriert ist, um ein erstes Bild eines ersten Scanbereichs, der dem ersten Scanort entspricht, und ein zweites Bild eines zweiten Scanbereichs, der dem zweiten Scanort entspricht, zu erzeugen.

2. System nach Anspruch 1, wobei der erste PET-Scanner (205) einen ersten PET-Detektorring enthält, und der zweite PET-Scanner (205) einen zweiten PET-Detektorring enthält.

3. System nach Anspruch 1, wobei
der erste PET-Scanner (205) und der zweite PET-Scanner (205) an der Antriebsvorrichtung (215) angebracht sind, und
die Antriebsvorrichtung (215) konfiguriert ist, um den ersten PET-Scanner (205) und den zweiten PET-Scanner (205) so anzutreiben, dass diese sich entlang einer axialen Richtung bewegen.

4. System nach Anspruch 1, weiter einen CT-Scanner umfassend, der konfiguriert ist, um CT-Scandaten zu erhalten, wobei der Prozessor (260) weiter konfiguriert ist, um auf Basis der CT-Scandaten ein Scout-Bild zu erzeugen.

5. System nach Anspruch 4, wobei
das Scout-Bild einen ersten Identifikator und einen zweiten Identifikator enthält, und
die Steuereinheit (255) konfiguriert ist, um den ersten Scanort und den zweiten Scanort durch Erhalten eines Orts des ersten Identifikators und eines Orts des zweiten Identifikators zu bestimmen.

6. System nach Anspruch 1, wobei
der erste PET-Scanner (205) konfiguriert ist, um die ersten Scandaten zu einem oder mehreren ersten Zeitpunkten zu erhalten, der zweite PET-Scanner (205) konfiguriert ist, um die zweiten Scandaten zu einem oder mehreren zweiten Zeitpunkten zu erhalten,
der Prozessor (260) konfiguriert ist, um auf Basis der ersten Scandaten zu dem einen oder den mehreren ersten Zeitpunkten dynamische Bildgebung am ersten Scanbereich durchzuführen, und der Prozessor (260) konfiguriert ist, um auf Basis der zweiten Scandaten zu dem einen oder den mehreren zweiten Zeitpunkten dynamische Bildgebung am zweiten Scanbereich durchzuführen.

7. System nach Anspruch 6, wobei der Prozessor (260) konfiguriert ist, um auf Basis der dynamischen Bildgebung den Metabolismus eines Mittels im ersten Scanbereich und dem zweiten Scanbereich zu bestimmen, und der Metabolismus sich mit der Zeit verändert.

8. System nach Anspruch 1, wobei der Prozessor (260) konfiguriert ist, um auf Basis von Scandaten, die vom ersten PET-Scanner (205) und dem zweiten PET-Scanner (205) erhalten wurden, ein drittes Bild eines dritten Scanbereichs zu erzeugen, wobei sich der dritte Scanbereich zwischen dem ersten Scanbereich und dem zweiten Scanbereich befindet.

9. System nach Anspruch 8, wobei, um auf Basis der Scandaten, die vom ersten PET-Scanner (205) und dem zweiten PET-Scanner (205) erhalten wurden, das dritte Bild des dritten Scanbereichs zu erzeugen, der Prozessor (260) weiter konfiguriert ist, um:
dritte Scandaten durch sowohl den ersten PET-Scanner (205) als auch den zweiten PET-Scanner (205) zu erhalten; und
auf Basis der dritten Scandaten das dritte Bild des dritten Scanbereichs zu erzeugen.

10. System nach Anspruch 8, wobei der Prozessor (260) konfiguriert ist, um durch Zusammenfügen von Bildern des ersten Scanbereichs, des zweiten Scanbereichs und des dritten Scanbereichs, die auf Basis der vom ersten PET-Scanner (205) und dem zweiten PET-Scanner (205) erhaltenen Scandaten erzeugt werden, ein vollständiges Bild zu erzeugen.

11. System nach Anspruch 1, wobei
der erste PET-Scanner (205) konfiguriert ist, um einen ersten Scanparameter zu erhalten und den ersten Scanbereich gemäß dem ersten Scanparameter zu scannen; und
der zweite PET-Scanner (205) konfiguriert ist, um einen zweiten Scanparameter zu erhalten und den zweiten Scanbereich gemäß dem zweiten Scanparameter zu scannen, wobei sich der erste Scanparameter vom zweiten Scanparameter unterscheidet.

12. System für medizinische Bildgebung, umfassend:
einen Bildsensor, der konfiguriert ist, um Bilddaten zu erzeugen, wobei es sich bei dem Bildsensor um eine Kamera handelt;
eine bildgebende Einrichtung (110), mit:
einem ersten PET-Scanner (205),
einem zweiten PET-Scanner (205), und
einer Antriebsvorrichtung (215); und
eine Rechenvorrichtung (120), mit:
einer Steuereinheit (255), die konfiguriert ist, um auf Basis der erzeugten Bilddaten vom Bildsensor einen ersten Scanort und einen zweiten Scanort zu bestimmen, und
einem Prozessor (260), wobei:
der erste PET-Scanner (205) am ersten Scanort angebracht ist,
die Antriebsvorrichtung (215) konfiguriert ist, um den zweiten PET-Scanner (205) so anzutreiben, dass dieser sich zum zweiten Scanort bewegt,
der erste PET-Scanner (205) und der zweite PET-Scanner (205) jeweils konfiguriert sind, um erste Scandaten und zweite Scandaten zu erhalten, und
der Prozessor (260) konfiguriert ist, um ein erstes Bild eines ersten Scanbereichs, der dem ersten Scanort entspricht, und ein zweites Bild eines zweiten Scanbereichs, der dem zweiten Scanort entspricht, zu erzeugen.

13. Verfahren für medizinische Bildgebung unter Verwendung des Systems nach Anspruch 1 oder Anspruch 12, umfassend:
Erhalten eines ersten PET-Scanparameters und eines zweiten PET-Scanparameters;
Bestimmen eines ersten Scanorts und eines zweiten Scanorts auf Basis von erzeugten Bilddaten von einem Bildsensor, wobei es sich bei dem Bildsensor um eine Kamera handelt;
Erzeugen erster Scandaten durch Scannen eines ersten Scanbereichs, der dem ersten Scanort entspricht, gemäß dem ersten PET-Scanparameter;
Erzeugen zweiter Scandaten durch Scannen eines zweiten Scanbereichs, der dem zweiten Scanort entspricht, gemäß dem zweiten PET-Scanparameter;
Erzeugen eines ersten Bildes des ersten Scanbereichs auf Basis der ersten Scandaten; und
Erzeugen eines zweiten Bildes des zweiten Scanbereichs auf Basis der zweiten Scandaten.

14. Nichtflüchtiges computerlesbares Medium, das Anweisungen speichert, die geeignet sind, das Verfahren nach Anspruch 13 durchzuführen.

## Revendications

1. Système d'imagerie médicale comprenant :
un capteur d'image configuré pour générer des données d'images, dans lequel le capteur d'image est une caméra ;
un appareil d'imagerie (110) incluant :
un premier tomographe (205), un second tomographe (205) et un dispositif d'entraînement (215) ; et
un dispositif informatique (120) incluant :
un dispositif de commande (255) configuré pour déterminer un premier emplacement de balayage et un second emplacement de balayage sur la base des données d'images générées par le capteur d'image, et
un processeur (260), dans lequel,
le dispositif d'entraînement (215) est configuré pour entraîner le premier tomographe (205) et
le second tomographe (205) pour qu'ils se déplacent vers le premier emplacement de balayage et le second emplacement de balayage, respectivement,
le premier tomographe (205) et le second tomographe (205) sont configurés pour obtenir des premières données de balayage et des deuxièmes données de balayage, respectivement, et
le processeur (260) est configuré pour générer une première image d'une première zone de balayage correspondant au premier emplacement de balayage et une deuxième image d'une deuxième zone de balayage correspondant au second emplacement de balayage.

2. Système selon la revendication 1, dans lequel le premier tomographe (205) inclut un premier anneau détecteur PET et le second tomographe (205) inclut un second anneau détecteur PET.

3. Système selon la revendication 1, dans lequel
le premier tomographe (205) et le second tomographe (205) sont montés sur le dispositif d'entraînement (215), et
le dispositif d'entraînement (215) est configuré pour entraîner le premier tomographe (205) et le second tomographe (205) pour qu'ils se déplacent le long d'une direction axiale.

4. Système selon la revendication 1, comprenant en outre un tomodensitomètre configuré pour obtenir des données de tomodensitométrie, dans lequel le processeur (260) est en outre configuré pour générer une image de repérage sur la base des données de tomodensitométrie.

5. Système selon la revendication 4, dans lequel
l'image de repérage inclut un premier identificateur et un second identificateur, et
le dispositif de commande (255) est configuré pour déterminer le premier emplacement de balayage et le second emplacement de balayage en obtenant un emplacement du premier identificateur et un emplacement du second identificateur.

6. Système selon la revendication 1, dans lequel
le premier tomographe (205) est configuré pour obtenir les premières données de balayage à un ou plusieurs premiers moments, le second tomographe (205) est configuré pour obtenir les deuxièmes données de balayage à un ou plusieurs seconds moments,
le processeur (260) est configuré pour réaliser une imagerie dynamique sur la première zone de balayage sur la base des premières données de balayage aux un ou plusieurs premiers moments, et le processeur (260) est configuré pour réaliser une imagerie dynamique sur la deuxième zone de balayage sur la base des deuxièmes données de balayage aux un ou plusieurs seconds moments.

7. Système selon la revendication 6, dans lequel le processeur (260) est configuré pour déterminer un métabolisme d'un agent dans la première zone de balayage et la deuxième zone de balayage sur la base de l'imagerie dynamique, et le métabolisme change au fil du temps.

8. Système selon la revendication 1, dans lequel le processeur (260) est configuré pour générer une troisième image d'une troisième zone de balayage sur la base de données de balayage obtenues par le premier tomographe (205) et le second tomographe (205), la troisième zone de balayage étant entre la première zone de balayage et la deuxième zone de balayage.

9. Système selon la revendication 8, dans lequel pour générer la troisième image de la troisième zone de balayage sur la base des données de balayage obtenues par le premier tomographe (205) et le second tomographe (205), le processeur (260) est en outre configuré pour :
obtenir des troisièmes données de balayage par le biais à la fois du premier tomographe (205) et du second tomographe (205) ; et
générer la troisième image de la troisième zone de balayage sur la base des troisièmes données de balayage.

10. Système selon la revendication 8, dans lequel le processeur (260) est configuré pour générer une image complète en assemblant des images de la première zone de balayage, la deuxième zone de balayage et la troisième zone de balayage qui sont générées sur la base des données de balayage obtenues par le premier tomographe (205) et le second tomographe (205).

11. Système selon la revendication 1, dans lequel,
le premier tomographe (205) est configuré pour obtenir un premier paramètre de balayage et pour balayer la première zone de balayage conformément au premier paramètre de balayage ; et
le second tomographe (205) est configuré pour obtenir un second paramètre de balayage et pour balayer la deuxième zone de balayage conformément au second paramètre de balayage, le premier paramètre de balayage étant différent du second paramètre de balayage.

12. Système d'imagerie médicale comprenant :
un capteur d'image configuré pour générer des données d'images, dans lequel le capteur d'image est une caméra ;
un appareil d'imagerie (110) incluant :
un premier tomographe (205),
un second tomographe (205), et
un dispositif d'entraînement (215) ; et
un dispositif informatique (120) incluant :
un dispositif de commande (255) configuré pour déterminer un premier emplacement de balayage et un second emplacement de balayage sur la base des données d'images générées par le capteur d'image, et
un processeur (260), dans lequel :
le premier tomographe (205) est monté au premier emplacement de balayage,
le dispositif d'entraînement (215) est configuré pour entraîner le second tomographe (205) pour qu'il se déplace vers le second emplacement de balayage,
le premier tomographe (205) et le second tomographe (205) sont respectivement configurés pour obtenir des premières données de balayage et des deuxièmes données de balayage, et
le processeur (260) est configuré pour générer une première image d'une première zone de balayage correspondant au premier emplacement de balayage et une deuxième image d'une deuxième zone de balayage correspondant au second emplacement de balayage.

13. Procédé d'imagerie médicale utilisant le système selon la revendication 1 ou la revendication 12 comprenant :
une obtention d'un premier paramètre de tomographie et d'un second paramètre de tomographie ;
une détermination d'un premier emplacement de balayage et d'un second emplacement de balayage sur la base de données d'images générées par un capteur d'image, dans lequel le capteur d'image est une caméra ;
une génération de premières données de balayage en balayant une première zone de balayage correspondant au premier emplacement de balayage conformément au premier paramètre de tomographie ;
une génération de deuxièmes données de balayage en balayant une deuxième zone de balayage correspondant au second emplacement de balayage conformément au second paramètre de tomographie ;
une génération d'une première image de la première zone de balayage sur la base des premières données de balayage ; et
une génération d'une deuxième image de la deuxième zone de balayage sur la base des deuxièmes données de balayage.

14. Support non transitoire lisible par ordinateur stockant des instructions adaptées pour réaliser le procédé selon la revendication 13.
